(19) Europäisches Patentamt
European Patent Office
Office européen des brevets

(11) EP 1 051 522 B1

(12) **EUROPÄISCHE PATENTSCHRIFT**

(45) Veröffentlichungstag und Bekanntmachung des Hinweises auf die Patenterteilung:
**25.05.2011 Patentblatt 2011/21**

(21) Anmeldenummer: **99910190.0**

(22) Anmeldetag: **03.02.1999**

(51) Int Cl.:
***C12Q 1/68*** *(2006.01)*

(86) Internationale Anmeldenummer:
**PCT/EP1999/000716**

(87) Internationale Veröffentlichungsnummer:
**WO 1999/040221 (12.08.1999 Gazette 1999/32)**

(54) **VERFAHREN ZUR QUANTITATIVEN BESTIMMUNG VON TUMORZELLEN IN EINER KÖRPERFLÜSSIGKEIT UND DAZU GEEIGNETE TESTKITS**

METHOD FOR QUANTITATIVELY ANALYZING TUMOR CELLS IN A BODY FLUID AND TEST KITS SUITED THEREFOR

PROCEDE POUR LA DETERMINATION QUANTITATIVE DE CELLULES TUMORALES DANS UN FLUIDE CORPOREL ET TROUSSES D'ESSAI APPROPRIEES A CET EFFET

(84) Benannte Vertragsstaaten:
**AT BE CH CY DE DK ES FI FR GB GR IE IT LI LU MC NL PT SE**

(30) Priorität: **04.02.1998 DE 19804372**

(43) Veröffentlichungstag der Anmeldung:
**15.11.2000 Patentblatt 2000/46**

(73) Patentinhaber: **Dahm, Michael W., Dr. Dr.
81677 München (DE)**

(72) Erfinder:
- **DAHM, Michael, W.
D-81677 München (DE)**
- **PHELPS, Robert, C.
D-61118 Bad Vilbel (DE)**
- **BROCKMEYER, Carsten
D-85354 Freising (DE)**

(74) Vertreter: **Keller, Günter
Lederer & Keller
Patentanwälte
Unsöldstrasse 2
80538 München (DE)**

(56) Entgegenhaltungen:
**WO-A-96/01835 WO-A-97/18322
WO-A-98/14592 WO-A-98/37181
WO-A-98/59040 GB-A- 2 317 891**

- **MEYERSON M ET AL: "hEST2, THE PUTATIVE HUMAN TELOMERASE CATALYTIC SUBUNIT GENE, IS UP -REGULATED IN TUMOR CELLS AND DURING IMMORTALIZATION" CELL, Bd. 90, Nr. 4, 22. August 1997 (1997-08-22), Seiten 785-795, XP002056804 ISSN: 0092-8674 in der Anmeldung erwähnt**
- **NAKAMURA T M ET AL: "TELOMERASE CATALYTIC SUBUNIT HOMOLOGS FROM FISSION YEAST AND HUMAN" SCIENCE, Bd. 277, 15. August 1997 (1997-08-15), Seiten 955-959, XP002056803 ISSN: 0036-8075 in der Anmeldung erwähnt**
- **KIM N W ET AL: "SPECIFIC ASSOCIATION OF HUMAN TELOMERASE ACTIVITY WITH IMMORTAL CELLS AND CANCER" SCIENCE, Bd. 266, 23. Dezember 1994 (1994-12-23), Seiten 2011-2015, XP002028668 ISSN: 0036-8075 in der Anmeldung erwähnt**
- **BLASCO M A ET AL: "DIFFERENTIAL REGULATION OF TELOMERASE ACTIVITY AND TELOMERASE RNA DURING MULTI-STAGE TUMORIGENESIS" NATURE GENETICS, Bd. 12, Nr. 2, 1. Februar 1996 (1996-02-01), Seiten 200-204, XP000673791 ISSN: 1061-4036**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 051 522 B1

SEQUENZPROTOKOLL

(1) ALLGEMEINE INFORMATION:
ANMELDER:

(A) NAME: Dr. Dr. Michael Dahm
(B) STRASSE: Gleimstr.2
(C) ORT: München
(E) LAND: Germany
(F) POSTLEITZAHL: 81677

ANMELDETITEL:
Verfahren zur quantitativen Bestimmung von Tumorzellen in einer Körperflüssigkeit und dazu geeignete Testkits
ANZAHL DER SEQUENZEN: 10
COMPUTER-LESBARE FORM:

(A) DATENTRÄGER: Floppy Disk
(B) COMPUTER: IBM PC compatible
(C) BETRIEBSSYSTEM: PC-DOS/MS-DOS
(D) SOFTWARE: PADAT Sequenzmodul Version 1.0

(2) INFORMATION ZU SEQ ID NO: 1:

(i) SEQUENZ CHARAKTERISTIKA:

(A) LÄNGE: 34 Basenpaare
(B) ART: Nukleinsäure
(C) STRANGFORM: Einzel
(D) TOPOLOGIE: linear

(ii) ART DES MOLEKÜLS: Genom-DNA
(xi) SEQUENZBESCHREIBUNG: SEQ ID NO: 1:
CTACCGGAAG AGTGTCTGGA GCAAGTTGCA AAGC 34

(2) INFORMATION ZU SEQ ID NO: 2:

(i) SEQUENZ CHARAKTERISTIKA:

(A) LÄNGE: 30 Basenpaare
(B) ART: Nukleinsäure
(C) STRANGFORM: Einzel
(D) TOPOLOGIE: linear

(ii) ART DES MOLEKÜLS: Genom-DNA
(xi) SEQUENZBESCHREIBUNG: SEQ ID NO: 2:
GGCATACCGA CGCACGCAGT ACGTGTTCTG 30

(2) INFORMATION ZU SEQ ID NO: 3:

(i) SEQUENZ CHARAKTERISTIKA:

(A) LÄNGE: 20 Basenpaare
(B) ART: Nukleinsäure
(C) STRANGFORM: Einzel
(D) TOPOLOGIE: linear

(ii) ART DES MOLEKÜLS: Genom-DNA
(xi) SEQUENZBESCHREIBUNG: SEQ ID NO: 3:
ACCCAGAGGT TCTTTGAGTC 20

(2) INFORMATION ZU SEQ ID NO: 4:

(i) SEQUENZ CHARAKTERISTIKA:

(A) LÄNGE: 20 Basenpaare
(B) ART: Nukleinsäure
(C) STRANGFORM: Einzel
(D) TOPOLOGIE: linear

(ii) ART DES MOLEKÜLS: Genom-DNA
(xi) SEQUENZBESCHREIBUNG: SEQ ID NO: 4:
TCTGATAGGC AGCCTGCACT - 20

(2) INFORMATION ZU SEQ ID NO: 5:

(i) SEQUENZ CHARAKTERISTIKA:

(A) LÄNGE: 24 Basenpaare
(B) ART: Nukleinsäure
(C) STRANGFORM: Einzel
(D) TOPOLOGIE: linear

(ii) ART DES MOLEKÜLS: Genom-DNA
(xi) SEQUENZBESCHREIBUNG: SEQ ID NO: 5:
GATGATGATA TCGCCGCGCT CGTC 24

(2) INFORMATION ZU SEQ ID NO: 6:

(i) SEQUENZ CHARAKTERISTIKA:

(A) LÄNGE: 25 Basenpaare
(B) ART: Nukleinsäure
(C) STRANGFORM: Einzel
(D) TOPOLOGIE: linear

(ii) ART DES MOLEKÜLS: Genom-DNA
(xi) SEQUENZBESCHREIBUNG: SEQ ID NO: 6:
CTCAAACATG ATCTGGGTCA TCTTC 25

(2) INFORMATION ZU SEQ ID NO: 7:

(i) SEQUENZ CHARAKTERISTIKA:

(A) LÄNGE: 28 Basenpaare
(B) ART: Nukleinsäure
(C) STRANGFORM: Einzel
(D) TOPOLOGIE: linear

(ii) ART DES MOLEKÜLS: Genom-DNA

(xi) SEQUENZBESCHREIBUNG: SEQ ID NO: 7:
GAGGTCGCTG TGTTTGAGCC ATCAGAAG 28

(2) INFORMATION ZU SEQ ID NO: 8:

(i) SEQUENZ CHARAKTERISTIKA:

(A) LÄNGE: 27 Basenpaare
(B) ART: Nukleinsäure
(C) STRANGFORM: Einzel
(D) TOPOLOGIE: linear

(ii) ART DES MOLEKÜLS: Genom-DNA
(xi) SEQUENZBESCHREIBUNG: SEQ ID NO: 8:
GATCTCATAG AGGATGGTGG CAGACAG 27

(2) INFORMATION ZU SEQ ID NO: 9:

(i) SEQUENZ CHARAKTERISTIKA:

(A) LÄNGE: 24 Basenpaare
(B) ART: Nukleinsäure
(C) STRANGFORM: Einzel
(D) TOPOLOGIE: linear

(ii) ART DES MOLEKÜLS: Genom-DNA
(xi) SEQUENZBESCHREIBUNG: SEQ ID NO: 9:
CGTTCTGGCT CCCACGACGT AGTC 24

(2) INFORMATION ZU SEQ ID NO: 10:

(i) SEQUENZ CHARAKTERISTIKA:

(A) LÄNGE: 4015 Basenpaare
(B) ART: Nukleinsäure
(C) STRANGFORM: Einzel
(D) TOPOLOGIE: linear

(ii) ART DES MOLEKÜLS: Genom-DNA
(xi) SEQUENZBESCHREIBUNG: SEQ ID NO: 10:

```
GCAGCGCTGC GTCCTGCTGC GCACGTGGGA AGCCCTGGCC CCGGCCACCC CCGCGATGCC   60
GCGCGCTCCC CGCTGCCGAG CCGTGCGCTC CCTGCTGCGC AGCCACTACC GCGAGGTGCT  120
GCCGCTGGCC ACGTTCGTGC GGCGCCTGGG GCCCCAGGGC TGGCGGCTGG TGCAGCGCGG  180
GGACCCGGCG GCTTTCCGCG CGCTGGTGGC CCAGTGCCTG GTGTGCGTGC CCTGGGACGC  240
ACGGCCGCCC CCCGCCGCCC CCTCCTTCCG CCAGGTGTCC TGCCTGAAGG AGCTGGTGGC  300
CCGAGTGCTG CAGAGGCTGT GCGAGCGCGG CGCGAAGAAC GTGCTGGCCT TCGGCTTCGC  360
GCTGCTGGAC GGGGCCCGCG GGGGCCCCCC CGAGGCCTTC ACCACCAGCG TGCGCAGCTA  420
CCTGCCCAAC ACGGTGACCG ACGCACTGCG GGGGAGCGGG GCGTGGGGGC TGCTGCTGCG  480
CCGCGTGGGC GACGACGTGC TGGTTCACCT GCTGGCACGC TGCGCGCTCT TTGTGCTGGT  540
GGCTCCCAGC TGCGCCTACC AGGTGTGCGG GCCGCCGCTG TACCAGCTCG GCGCTGCCAC  600
TCAGGCCCGG CCCCCGCCAC ACGCTAGTGG ACCCCGAAGG CGTCTGGGAT GCGAACGGGC  660
CTGGAACCAT AGCGTCAGGG AGGCCGGGGT CCCCCTGGGC CTGCCAGCCC CGGGTGCGAG  720
GAGGCGCGGG GGCAGTGCCA GCCGAAGTCT GCCGTTGCCC AAGAGGCCCA GGCGTGGCGC  780
TGCCCCTGAG CCGGAGCGGA CGCCCGTTGG GCAGGGGTCC TGGGCCCACC CGGGCAGGAC  840
GCGTGGACCG AGTGACCGTG GTTTCTGTGT GGTGTCACCT GCCAGACCCG CCGAAGAAGC  900
CACCTCTTTG GAGGGTGCGC TCTCTGGCAC GCGCCACTCC CACCCATCCG TGGGCCGCCA  960
GCACCACGCG GGCCCCCCAT CCACATCGCG GCCACCACGT CCCTGGGACA CGCCTTGTCC 1020
CCCGGTGTAC GCCGAGACCA AGCACTTCCT CTACTCCTCA GGCGACAAGG AGCAGCTGCG 1080
GCCCTCCTTC CTACTCAGCT CTCTGAGGCC CAGCCTGACT GGCGCTCGGA GGCTCGTGGA 1140
GACCATCTTT CTGGGTTCCA GGCCCTGGAT GCCAGGGACT CCCCGCAGGT TGCCCCGCCT 1200
GCCCCAGCGC TACTGGCAAA TGCGGCCCCT GTTTCTGGAG CTGCTTGGGA ACCACGCGCA 1260
```

GTGCCCCTAC GGGGTGCTCC TCAAGACGCA CTGCCCGCTG CGAGCTGCGG TCACCCCAGC 1320

AGCCGGTGTC TGTGCCCGGG AGAAGCCCCA GGGCTCTGTG GCGGCCCCCG AGGAGGAGGA 1380

CACAGACCCC CGTCGCCTGG TGCAGCTGCT CCGCCAGCAC AGCAGCCCCT GGCAGGTGTA 1440

CGGCTTCGTG CGGGCCTGCC TGCGCCGGCT GGTGCCCCCA GGCCTCTGGG GCTCCAGGCA 1500

CAACGAACGC CGCTTCCTCA GGAACACCAA GAAGTTCATC TCCCTGGGGA AGCATGCCAA 1560

GCTCTCGCTG CAGGAGCTGA CGTGGAAGAT GAGCGTGCGG GACTGCGCTT GGCTGCGCAG 1620

GAGCCCAGGG GTTGGCTGTG TTCCGGCCGC AGAGCACCGT CTGCGTGAGG AGATCCTGGC 1680

CAAGTTCCTG CACTGGCTGA TGAGTGTGTA CGTCGTCGAG CTGCTCAGGT CTTTCTTTTA 1740

TGTCACGGAG ACCACGTTTC AAAAGAACAG GCTCTTTTTC TACCGGAAGA GTGTCTGGAG 1800

CAAGTTGCAA AGCATTGGAA TCAGACAGCA CTTGAAGAGG GTGCAGCTGC GGGAGCTGTC 1860

GGAAGCAGAG GTCAGGCAGC ATCGGGAAGC CAGGCCCGCC CTGCTGACGT CCAGACTCCG 1920

CTTCATCCCC AAGCCTGACG GGCTGCGGCC GATTGTGAAC ATGGACTACG TCGTGGGAGC 1980

CAGAACGTTC CGCAGAGAAA AGAGGGCCGA GCGTCTCACC TCGAGGGTGA AGGCACTGTT 2040

CAGCGTGCTC AACTACGAGC GGGCGCGGCG CCCCGGCCTC CTGGGCGCCT CTGTGCTGGG 2100

CCTGGACGAT ATCCACAGGG CCTGGCGCAC CTTCGTGCTG CGTGTGCGGG CCCAGGACCC 2160

GCCGCCTGAG CTGTACTTTG TCAAGGTGGA TGTGACGGGC GCGTACGACA CCATCCCCCA 2220

GGACAGGCTC ACGGAGGTCA TCGCCAGCAT CATCAAACCC CAGAACACGT ACTGCGTGCG 2280

TCGGTATGCC GTGGTCCAGA AGGCCGCCCA TGGGCACGTC CGCAAGGCCT TCAAGAGCCA 2340

CGTCTCTACC TTGACAGACC TCCAGCCGTA CATGCGACAG TTCGTGGCTC ACCTGCAGGA 2400

GACCAGCCCG CTGAGGGATG CCGTCGTCAT CGAGCAGAGC TCCTCCCTGA ATGAGGCCAG 2460

CAGTGGCCTC TTCGACGTCT TCCTACGCTT CATGTGCCAC CACGCCGTGC GCATCAGGGG 2520

CAAGTCCTAC GTCCAGTGCC AGGGGATCCC GCAGGGCTCC ATCCTCTCCA CGCTGCTCTG 2580

CAGCCTGTGC TACGGCGACA TGGAGAACAA GCTGTTTGCG GGGATTCGGC GGGACGGGCT 2640

GCTCCTGCGT TTGGTGGATG ATTTCTTGTT GGTGACACCT CACCTCACCC ACGCGAAAAC 2700

CTTCCTCAGG ACCCTGGTCC GAGGTGTCCC TGAGTATGGC TGCGTGGTGA ACTTGCGGAA 2760

GACAGTGGTG AACTTCCCTG TAGAAGACGA GGCCCTGGGT GGCACGGCTT TTGTTCAGAT 2820

GCCGGCCCAC GGCCTATTCC CCTGGTGCGG CCTGCTGCTG GATACCCGGA CCCTGGAGGT 2880

GCAGAGCGAC TACTCCAGCT ATGCCCGGAC CTCCATCAGA GCCAGTCTCA CCTTCAACCG 2940
CGGCTTCAAG GCTGGGAGGA ACATGCGTCG CAAACTCTTT GGGGTCTTGC GGCTGAAGTG 3000
TCACAGCCTG TTTCTGGATT TGCAGGTGAA CAGCCTCCAG ACGGTGTGCA CCAACATCTA 3060
CAAGATCCTC CTGCTGCAGG CGTACAGGTT TCACGCATGT GTGCTGCAGC TCCCATTTCA 3120
TCAGCAAGTT TGGAAGAACC CCACATTTTT CCTGCGCGTC ATCTCTGACA CGGCCTCCCT 3180
CTGCTACTCC ATCCTGAAAG CCAAGAACGC AGGGATGTCG CTGGGGGCCA AGGGCGCCGC 3240
CGGCCCTCTG CCCTCCGAGG CCGTGCAGTG GCTGTGCCAC CAAGCATTCC TGCTCAAGCT 3300
GACTCGACAC CGTGTCACCT ACGTGCCACT CCTGGGGTCA CTCAGGACAG CCCAGACGCA 3360
GCTGAGTCGG AAGCTCCCGG GGACGACGCT GACTGCCCTG GAGGCCGCAG CCAACCCGGC 3420
ACTGCCCTCA GACTTCAAGA CCATCCTGGA CTGATGGCCA CCCGCCCACA GCCAGGCCGA 3480
GAGCAGACAC CAGCAGCCCT GTCACGCCGG GCTCTACGTC CCAGGGAGGG AGGGGCGGCC 3540
CACACCCAGG CCCGCACCGC TGGGAGTCTG AGGCCTGAGT GAGTGTTTGG CCGAGGCCTG 3600
CATGTCCGGC TGAAGGCTGA GTGTCCGGCT GAGGCCTGAG CGAGTGTCCA GCCAAGGGCT 3660
GAGTGTCCAG CACACCTGCC GTCTTCACTT CCCCACAGGC TGGCGCTCGG CTCCACCCCA 3720
GGGCCAGCTT TTCCTCACCA GGAGCCCGGC TTCCACTCCC CACATAGGAA TAGTCCATCC 3780
CCAGATTCGC CATTGTTCAC CCCTCGCCCT GCCCTCCTTT GCCTTCCACC CCCACCATCC 3840
AGGTGGAGAC CCTGAGAAGG ACCCTGGGAG CTCTGGGAAT TTGGAGTGAC CAAAGGTGTG 3900
CCCTGTACAC AGGCGAGGAC CCTGCACCTG GATGGGGGTC CCTGTGGGTC AAATTGGGGG 3960
GAGGTGCTGT GGGAGTAAAA TACTGAATAT ATGAGTTTTT CAGTTTTGAA AAAAA 4015

## Beschreibung

Die Erfindung betrifft ein Verfahren zur Quantifizierung von Tumorzellen in einer Körperflüssigkeit, bei dem zuerst die zu untersuchende Probe einem Verfahren zur An- bzw. Abreicherung von Tumorzellen unterzogen wird und an den angereicherten bzw. abgereicherten Tumorzellen eine Reaktion durchgeführt wird, bei der die für die katalytische Untereinheit der Telomerase kodierende mRNA spezifisch amplifiziert, und anschließend die Menge an amplifizierter Nukleinsäure quantitativ bestimmt wird sowie dazu geeignete Testkits.

Nahezu alle soliden malignen Tumore haben das Potential zur Metastasenbildung. Der Prozeß der Metastasierung beinhaltet die Aussaat maligner Zellen als Mikrometastasen, zumeist auf dem Blut- bzw. Lymphweg in entfernte Organe und die Ausbildung autonomer Tochtergeschwülste. Das Ausmaß der Filiarisierung bestimmt die Prognose eines Tumorleidens.

Die Ansprüche von Tumorvorsorge- oder Nachsorgeprogrammen liegen in der Früherkennung von Primärtumoren bzw. eines Rezidivs oder einer Metastasierung noch bevor Metastasen klinisch manifest werden. Dieses Ziel kann bislang mit den verfügbaren apparativen Techniken nicht zufriedenstellend erfüllt werden, insbesondere gibt es immer noch eine diagnostische Grauzone zwischen dem Nachweis zirkulierender Tumorzellen und beginnender Metastasenbildung in Organen. Durch die Frühdiagnose zirkulierender maligner Zellen z.B. im peripheren Blut eines Tumornachsorgepatienten könnte frühzeitig, d.h. noch vor einer manifesten Organmetastasierung, eine möglicherweise kurative Immunmodulations- oder Polychemotherapie eingeleitet werden. Die Quantifizierung der Tumorzellen im peripheren Blut vor und nach der Therapie stellt dabei eine wichtige Kontrolle dar.

Die Lebensdauer eukaryontischer Zellen wird entsprechend der Telomer-Hypothese durch die Länge der Termini der chromosomalen DNA, der Telomere, bestimmt. Telomere spielen deshalb nach dieser Theorie die Rolle einer mitotischen Uhr. Bedingt durch den Replikationsmechanismus der DNA-Polymerasen werden die Telomere nach jeder Zellteilung um die Länge des Replikationsprimers verkürzt. Dadurch werden die Chromosomen nach jeder Zellteilung kürzer und erreichen nach einer bestimmten Anzahl von Zellteilungen eine kritische Länge. Die Zellen gehen dann in eine seneszente Phase über, in der sie sich nicht mehr teilen und schließlich absterben. In einigen Fällen kann jedoch dieser Regulationsmechanismus durch ein Ribonukleoprotein, die Telomerase, übergangen werden und die Zellen werden immortal. Die Telomerase synthetisiert die bei der Replikation verlorengegangenen Telomersequenzen an das 5'-Ende der Chromosomen, wobei die RNA-Komponente des Proteins (human Telomerase RNA component, hTR) als Matrize dient und ein Teil der Proteinkomponente die katalytische Untereinheit (human Telomerase Reverse Transcriptase, hTRT) bildet.

Zu den Zellen mit aktiver Telomerase und unbegrenzter Lebensdauer im Menschen gehören vor allem die Keimzellen und hämatopoetischen Stammzellen, die sich während der gesamten Lebenszeit eines Individuums teilen können. Darüber hinaus werden auch in aktivierten B- und T-Lymphozyten des Menschen erhöhte Telomerase-Aktivitäten gefunden. Neben dieser normalen physiologischen Rolle der Telomerase kann in etwa 90-95% aller menschlichen Tumorgeweben eine erhöhte Telomerase-Aktivität gefunden werden. Daher kann die Telomerase-Aktivität von Tumorzellen die Grundlage für ein Nachweissystem für disseminierte zirkulierende Tumorzellen in Körperflüssigkeiten bilden, die potentiell zu Metastasen führen können.

Die Telomerase ist ein Ribonukleoprotein mit reverser Transkriptaseaktivität [Shippen-Lentz et al. (1990), Science 247: 546], das als Matrize eine integrale RNA-Sequenz zur unabhängigen DNA-Synthese benutzt [Greider et al. (1989). Nature 337: 331], mit der neue telomere DNA an die Enden der Chromosomen synthetisiert werden. Telomere bestehen aus hoch konservierten (TTAGGG)n Tandemsequenzen von ca. 5-15 Kilobasen (kb) Länge/Zellgenom und haben die Aufgabe die Chromosomen an der Kernmembran zu stabilisieren und schützen die kodierende genomische DNA vor unkontrollierter Rekombination und Degradation [Mehle et al. (1994). Cancer Res 54: 236]. Während in den niederen Eukaryonten ein dynamisches Gleichgewicht zwischen Verkürzung der Chromosomenenden und der de novo Synthese von telomeren Sequenzen durch die Telomerase postuliert wird, zeigen normale humane somatische Zellen eine niedrige oder nicht nachweisbare Telomeraseaktivität. Darüber hinaus ist die Telomerase im Gegensatz zu anderen DNA Enzymen nicht wachstumsreguliert, denn keine der aktiv proliferierenden Zellkulturen zeigte nachweisbare Telomeraseaktivität. Einzig Keimzellen und fast alle Tumorzellinien [Ohyashiki et al. (1994). Cancer Genet Cytogenet 78: 64; Rogalla et al. (1994). Cancer Genet Cytogenet 77: 19; Schwartz et al. (1995). Cancer 75: 1094] und Tumorgewebe (Lunge, [Hiyama et al. (1995). Oncogene 10: 937; Shirotani et al. (1994). Lung Cancer 11: 29], Nieren [Mehle et al. (1994). Cancer Res 54: 236], Ovarien [Chadeneau et al. (1995). Cancer Res 55: 2533] und Blut [Counter et al. (1995). Blood 85: 2315]) zeigen meßbare Telomeraseaktivität und eine konstante Telomerlänge, die durch eine unendliche Zahl von Zellteilungen hindurch beibehalten wird. Daher kann die Aktivierung der Telomerase mit der damit verbundenen Stabilisierung der Telomerlängen als kritischer Schritt in Richtung Immortalisierung von somatischen Zellen gewertet werden.

Feng et al. gelang die Klonierung der integralen RNA-Sequenz der humanen Telomerase (hTR), die auf dem distalen Segment (q) von Chromosom 3 kodiert wird. Die Autoren konnten mittels kompetitiver Polymerase-Kettenreaktion (PCR) eine signifikante Erhöhung der Telomeraseexpression in Tumorgeweben sowie in den Keimgeweben gegenüber normalen somatischen Zellen belegen [Feng et al. (1995), Science 269: 1236]. Ein Antisense-Konstrukt der hTR-Sequenz verursachte den Zelltod (Apoptose) in transfizierten HeLA-Zellen. Diese Daten belegen die stringente Repression der Telomerase in somatischen Geweben als auch die Tatsache, daß malignes Wachstum von der Präsenz immortaler Zellen und von der Aktivierung der Telomerase abhängig ist.

Nakamura et al. charakterisierten 1997 einen Proteinbestandteil der katalytischen Untereinheit der menschlichen Telomerase. Im Vergleich mit der RNA-Komponente der menschlichen Telomerase (hTR) korreliert die für die katalytische Untereinheit. der menschlichen Telomerase (hTRT) kodierende mRNA wesentlich stärker mit der Telomeraseaktivität (Nakamura TM, Morin GB, Chapman KB, Weinrich SL, Andrews WH, Lingner J, Harley CB, Cech TR (1997): Telomerase catalytic subunit homologs from fission yeast and human. Science 277: 955-9) und ist damit besser zur Krebsdiagnostik geeignet. Meyerson et al. lokalisierten die hTRT auf dem menschlichen Chromosom 5p und bestätigten die starke Korrelation des hTRT mRNA-Nachweises mit der enzymatischen Aktivität der menschlichen Telomerase (Meyerson M, Counter CM, Eaton EN, Ellisen LW, Steiner P, Caddle SD, Ziaugra L, Beijersbergen RL, Davidoff MJ, Liu Q, Bacchetti S, Haber DA, Weinberg RA (1997): hEST2, the putative human telomerase catalytic subunit gene, is up-regulated in tumor cells and during immortalization. Cell 90: 785-95).

Die Standardmethode zur Bestimmung der katalytischen Aktivität der Telomerase ist z.Zt. der TRAP-Assay (Telomeric Repeat Amplification Protocol) [Kim et al. Science (1994) 266: 2011]. Dabei synthetisiert die im Zellextrakt vorhandene Telomerase Extensionsprodukte, die anschließend in einer Polymerase-Kettenreaktion (Polymerase Chain Reaction, PCR) amplifiziert werden. Eine densitometrische Auswertung der Amplifikationsprodukte erlaubt anschließend eine Quantifizierung der Telomerase-Aktivität. Die Sensitivität des TRAP-Assays wurde in Abhängigkeit des Probenmaterials, des Versuchslabors und des verwendeten Protokolls mit 4-100 Zellen/Ansatz angegeben. Da im TRAP-Assay der Rohextrakt von lysierten Zellen bzw. Geweben verwendet wird, ist diese Methode im hohen Maße störanfällig gegenüber Inhibitoren der in der PCR verwendeten DNA-Polymerase (Taq-Polymerase).

Der TRAP-Assay wird beispielsweise in der WO 98/02581 zur Diagnose von präkanzerösen oder kanzerösen Schäden des Cervix, Endocervix, der Vagina oder Vulva eingesetzt.

Die WO 97/18322 offenbart ein Verfahren zur Quantifizierung von Tumorzellen, bei dem zuerst mit der zu untersuchenden Probe eine Reaktion durchgeführt wird, bei der die RNA-Komponente der Telomerase spezifisch amplifiziert wird, und anschließend die Menge an amplifizierter Nukleinsäure quantitativ bestimmt wird.

Die WO 96/01835 offenbart ebenfalls ein Verfahren zum Nachweis der RNA-Komponente der Telomerase in Zellen oder Zellproben.

Trotz der bekannten Verfahren zum Nachweis von Tumorzellen besteht weiterhin ein Bedürfnis nach zuverlässigen und einfachen Verfahren zur Quantifizierung von Tumorzellen in Körperflüssigkeiten.

Aufgabe der vorliegenden Erfindung war daher, ein Verfahren zu entwickeln, mit dem man Tumorzellen in einer Körperflüssigkeit quantitativ bestimmen kann.

Die Erfindung betrifft daher ein Verfahren zur Quantifizierung von Tumorzellen in einer Körperflüssigkeit, bei dem zuerst die zu untersuchende Probe einem Verfahren zur An- bzw. Abreicherung von Tumorzellen unterzogen wird und an den angereicherten bzw. abgereicherten Tumorzellen eine Reaktion durchgeführt wird, bei der die für die katalytische Untereinheit der Telomerase kodierende mRNA spezifisch amplifiziert, und anschließend die Menge an amplifizierter Nukleinsäure quantitativ bestimmt wird sowie dazu geeignete Testkits. Zur Anreicherung der Tumorzellen wird ein Zellseparationsmedium mit der Körperflüssigkeit überschichtet und zentrifugiert, wobei das Zellseparationsmedium eine Dichte im Bereich von 1,060 bis < 1,065 g/ml aufweist.

Bei der Körperflüssigkeit, in der Tumorzellen quantifiziert werden sollen, kann es sich um jede menschliche oder tierische Körperflüssigkeit oder um eine Dispersion von Zellgewebe handeln. Hierbei handelt es sich beispielsweise um Blut, insbesondere peripheres Blut wie venöses oder arterielles Blut, Lymphe, Urin, Stuhl, Exsudate, Transudate, Spinalflüssigkeit, Samenflüssigkeit, Speichel, Flüssigkeiten aus natürlichen oder unnatürlichen Körperhöhlen, Knochenmark und dispergiertem Körpergewebe. Bei den Flüssigkeiten aus natürlichen Körperhöhlen kann es sich beispielsweise um seröse Flüssigkeiten wie Peritoneal- und Pleuraflüssigkeiten handeln, bei den Flüssigkeiten aus unnatürlichen Körperhöhlen kann es sich beispielsweise um Flüssigkeiten aus Zysten handeln.

Bevorzugte Körperflüssigkeiten sind Blut, Knochenmark, Lymphe, seröse Flüssigkeiten aus Körperhöhlen sowie Urin, wobei Blut und Urin besonders bevorzugt sind. Urin eignet sich insbesondere zur Anreicherung von Zellen von Blasentumoren.

Beispielsweise wird peripheres Blut durch Punktion einer Arterie, Vene oder Fingerkuppe dem Probanden entnommen und nach einem Anreicherungs- bzw. Abreicherungsverfahren, die in der Probe enthaltenen Tumorzellen in einen RNA-Lysispuffer der beispielsweise Harnstoff oder vorzugsweise Guanidinium Isothiocyanat enthält, überführt, um eventuell vorhandene RNasen zu denaturieren und die Nukleinsäuren aus den Zellen freizusetzen [siehe z. B. Chomczynski et al. (1987) Anal. Biochem. 162, 156]. Die Isolierung der Nukleinsäuren aus dem stark salzhaltigen Medium des RNA-Lysispuffers kann beispielsweise mittels Siliciumdioxid-Partikel erfolgen, an die sämtliche Nukleinsäuren binden können [Boom et al. (1990) J. Clin. Microbiol., 29, 495]. Danach werden die Partikel mit geeignetem Puffer mehrmals gewaschen und die gebundenen Nukleinsäuren eluiert. Anschließend ist es vorteilhaft die in der Probe eventuell vorhandene genomische DNA mittels RNasefreier DNase in einem geeigneten Puffer zu hydrolysieren, damit bei der späteren Amplifizierung der für die katalytische Untereinheit der Telomerase kodierenden mRNA keine falsch-positiven Ergebnisse bzw. ein zu großes Hintergrundrauschen durch falsche Amplifizierungssignale aufgrund eventuell noch vorhandener DNA entstehen. Anschließend erfolgt im allgemeinen eine Inaktivierung der DNase beispielsweise durch Phenolextraktion und/oder Hitzedenaturierung. Vor oder vorzugsweise nach Behandlung der Probe mit DNase kann vorteilhafterweise noch eine weitere Reinigung der in der Probe vorhandenen RNA beispielsweise mittels chromatographischer Methoden wie die IonenaustauschChromatographie vorzugsweise an Kieselgel erfolgen.

Zur Kontrolle, ob noch eventuell störende genomische DNA in der Probe vorhanden ist, kann anschließend eine Amplifizierungsreaktion mit den unten beschriebenen Telomerase-spezifischen Oligonukleotid-Primern durchgeführt werden, wobei die in der Probe vorhandene RNA vorher nicht in cDNA durch eine reverse Transkriptionsreaktion umgeschrieben wird. Nur für den Fall, daß die Probe frei ist von Telomerase-spezifischer DNA erfolgt keine Amplifikation mit der Folge, daß keine amplifizierte DNA gemessen werden kann.

Anschließend erfolgt eine Umschreibung der in der Probe vorhandenen RNA in cDNA im allgemeinen mit Hilfe der reversen Transkriptionsreaktion z. B. mit der AMV reversen Transkriptase. Das Verfahren ist allgemein bekannt und beispielsweise bei Sambrook et al., Molecular Cloning: A Laboratory Manual, New York Cold Spring Harbor Laboratory, 1989 beschrieben. In einer bevorzugten Ausführungsform der reversen Transkription kann auch eine thermostabile RNA-abhängige DNA Polymerase, wie in WO 90/07641 beschrieben, verwendet werden. Als Oligonukleotid-Primer für die reverse Transkriptase eignen sich beispielsweise vorteilhafterweise die unten beschriebenen Oligonukleotid-Primer oder Random Primer einer bestimmten Länge.

Die anschließende Amplifizierung kann beispielsweise mit einer DNA-Polymerase z. B. nach der Polymerase-Kettenreaktion (PCR) durchgeführt werden (siehe z. B. U.S. Patente Nr. 4,683,195; 4,683,202; 4,965,188) oder vorzugsweise mit einer RNA-Polymerase nach z. B. der isothermalen Nukleinsäuresequenz-basierenden Amplifikation (NASBA). In jedem Fall benötigt man für die Amplifizierung spezifische Oligonukleotid-Primer, die von der zu amplifizierenden Nukleinsäure abgeleitet sind. Bei der vorliegenden Erfindung kann jeder beliebige Sequenzabschnitt der für die katalytische Untereinheit der Telomerase kodierenden cDNA für die Synthese der Oligonukleotid-Primer verwendet werden. Vorzugsweise sind die Oligonukleotid-Primer ca. 20 bis ca. 40, vorzugsweise ca. 25 bis 35 Nukleotide lang. Das Amplifikationsprodukt ist im allgemeinen ca. 100 bis ca. 2000 Basen, vorzugsweise ca. 200 bis ca. 1500 Basen, insbesondere ca. 450 bis ca. 550 Basen lang. Bei dem erfindungsgemäßen Verfahren sind insbesondere folgende Oligonukleotid-Primer bevorzugt, die aus der Sequenz gemäß Abb. 1 abgeleitet wurden:

5' CTACCGGAAG AGTGTCTGGA GCAAGTTGCA AAGC 3' (hTRT1) und/oder
5' GGCATACCGA CGCACGCAGT ACGTGTTCTG 3' (hTRT2),

wobei hTRT1 und/oder hTRT2 gegebenenfalls zusätzlich eine Promotorsequenz für eine RNA-Polymerase enthalten können. Der Oligonukleotid-Primer hTRT1 entspricht dem 5'-Primer und hTRT2 dem 3'-Primer. Das Amplifikationsprodukt ist 513bp lang. Die Primer können beispielsweise synthetisch nach der Triestermethode hergestellt werden [Matteucci et al., (1981), J. Am. Chem. Soc., 103, 3185-3191]. Als DNA-Polymerase kann beispielsweise eine nicht-thermostabile DNA-Polymerase wie die T4 DNA Polymerase, T7 DNA Polymerase, E. coli Polymerase I oder das Klenow Fragment von E. coli oder vorzugsweise eine thermostabile DNA-Polymerase wie die Taq-Polymerase (siehe z. B. U.S. Patent Nr. 4,889,818) verwendet werden.

Das allgemeine Prinzip der PCR-Reaktion besteht nun darin, daß während mehrerer sich wiederholender Reaktionszyklen die DNA hitzedenaturiert wird und in Anwesenheit geeigneter Oligonukleotid-Primer mit gegenseitiger Orientierung der Einzelstrang mit Hilfe der DNA-Polymerase zum Doppelstrang wieder ergänzt wird. Danach wird der Zyklus wiederholt bis sich genügend DNA zur Quantifizierung nach einer der unten beschriebenen Methoden gebildet hat. Im allgemeinen sind ca. 20 bis ca. 40 Zyklen, vorzugsweise ca. 30 bis ca. 35 Zyklen ausreichend.

Bei der NASBA-Methode (auch 3SR-System genannt) wird zumindest ein Oligonukleotid-Primer, vorzugsweise hTRT2 verwendet, der einen Promotor für die RNA Polymerase, vorzugsweise für die T7 RNA Polymerase enthält [siehe z. B. Guatelli et al. (1990) Proc. Natl. Acad. Sci. USA, 87, 1874-1878 oder Kievits et al. (1991), J. Virol. Methods, 35, 273-286]. Zuerst wird, wie oben bereits näher beschrieben, die RNA mit Hilfe eines der oben beschriebenen Oligonukleotid-Primer und einer reversen Transkriptase, vorzugsweise der AMV reversen Transkriptase, in cDNA umgeschrieben. Das Reaktionsprodukt ist ein RNA:DNA-Doppelstrang, dessen RNA-Komponente anschließend mittels einer RNase, vorzugsweise der RNase H, zu einem DNA-Einzelstrang abgebaut wird. Unter Verwendung eines der oben beschriebenen Oligonukleotid-Primer wird der DNA-Einzelstrang mittels einer DNA Polymerase zum DNA-Doppelstrang ergänzt. Als DNA Polymerase eignet sich vorzugsweise wiederum die AMV reverse Transkriptase, da diese Transkriptase nicht nur eine RNA-abhängige DNA Polymeraseaktivität, sondern auch eine DNA-abhängige DNA Polymeraseaktivität besitzt. Das Reaktionsprodukt ist ein DNA-Doppelstrang, der den Promotor für z. B. die T7 RNA Polymerase enthält. Anschließend synthetisiert die RNA Polymerase wieder einen sogenannten "antisense" RNA-Strang und der Zyklus kann von neuem beginnen. Im allgemeinen sind ca. 20 bis ca. 40 Zyklen, vorzugsweise ca. 30 bis ca. 35 Zyklen ausreichend, um genügend Amplifikationsprodukt, vorzugsweise "antisense" RNA, für die anschließende Quantifizierung zu liefern.

Für die Quantifizierung der Amplifikationsprodukte können diese beispielsweise mit interkalierenden Fluoreszenzfarbstoffen wie beispielsweise Ethidiumbromid gefärbt und direkt z. B. in einem Agarose- oder Polyacrylamidgel unter ultraviolettem Licht sichtbar gemacht und quantifiziert werden.

Da die zu quantifizierenden Amplifikationsprodukte jedoch nur dann in direkter Korrelation zu der eingesetzten Menge RNA stehen, wenn sich die Amplifikations-Reaktion in einer linearen Phase befindet, ist es zum Zwecke der Quantifizierung notwendig, den Reaktionsverlauf der Amplifizierung zu messen. Dazu müssen die Reaktionsprodukte nach jedem Zyklus der Amplifizierung gemessen werden, wobei anhand des Reaktionsverlaufs der lineare Bereich bestimmt wird, in welchem dann mit hinreichender Sicherheit eine Quantifizierung vorgenommen werden kann. Es ist somit vorteilhaft, wenn die Amplifikationsprodukte bereits während der Amplifizierung markiert werden, und die Kinetik der Amplifikation kontinuierlich schon während des Amplifikationsvorganges gemessen wird. Dies wird beispielsweise bei der TaqMan™-PCR (Hoffmann-La Roche) mit Hilfe einer für das Amplifikationsprodukt spezifischen Sonde durchgeführt. Die Sonde ist an ihrem 3'- und 5'-Ende mit unterschiedlichen Fluoreszenzfarbstoffen ("Reporter" und "Quencher") markiert und liegt während der gesamten PCR im Reaktionsgefäß vor. Bedingt durch die 5'-3'-Exonukleaseaktivität der zur Amplifizierung verwendeten DNA-Polymerase (AmpiTaq™, Hoffmann-LaRoche) wird während der Verlängerung des DNA-Tochterstranges die Sonde hydrolysiert und dabei der Quencher vom Reporter getrennt. Dadurch wird der Quencheffekt aufgehoben und die vom "Reporter" emittierte charakteristische Fluoreszenz kann gemessen werden. Die Intensität dieser Fluoreszenz ist direkt proportional zu den PCR-Produkten, die pro Synthese-Zyklus amplifiziert wurden.

Als Markierungen eignen sich beispielsweise auch radioaktive Markierungen, Biotinmarkierungen, Fluoreszenz- oder Elektrochemolumineszenz(ECL)markierungen. Die Markierungen tragen in der Regel die Nukleotide als Ausgangsstoffe für die Amplifizierung durch die DNA- oder RNA-Polymerase. Die radioaktiv markierten Amplifikationsprodukte (z. B. PCR- oder NASBA-Produkte) können durch Messung der Radioaktivität, die Biotin-markierten Amplifikationsprodukte über einen Avidin- oder Streptavidin-tragenden Farbstoff, die fluoreszenzmarkierten Amplifikationsprodukte mit einem Fluorometer und die elektrochemolumineszenz-markierten Amplifikationsprodukte mit einem ECL-Detektor nachgewiesen werden. Die am meisten bevorzugte Nachweismethode ist jedoch die automatische in vitro Hybridisierung, z.B. wie bei der TaqMan™-PCR, schon während des Amplifikationsvorganges.

Im Unterschied zur "Echt-Zeit"-Quantifizierung von PCR-Produkten wie bei der TaqMan™-PCR ist es auch möglich PCR-Produkte mit Hilfe von Verdünnungsreihen zu quantifizieren. Dabei wird die RNA bzw. die entsprechende cDNA in verschiedenen Verdünnungsstufen eingesetzt. Dadurch erhält man ähnlich wie bei der "Echt-Zeit"-Quantifizierung eine sigmoidale Reaktionskinetik bei der wiederum im linearen Bereich die Amplifikationsprodukte proportional zum Ausgangsmaterial vorliegen. Der Nachweis der PCR-Produkte wird mit Hilfe von Fluoreszenzfarbstoff-markierten oder radioaktiv-markierten Desoxynukleotiden durchgeführt, die von der DNA-Polymerase während der Amplifizierung direkt in die PCR-Produkte eingebaut werden. Desweiteren werden Oligonukleotide (sog. Primer, die bei der PCR zur Initialisierung der Synthese des Tochterstranges notwendig sind und nach der PCR in allen Amplifikationsprodukten am 5'-und 3'-Ende der DNA eingebaut vorliegen) mit bestimmten Molekülen markiert (beispielsweise Biotin), um die Amplifikationsprodukte nach der PCR mit Hilfe von spezifischen Antikörpern oder Streptavidin zu immobilisieren. Diese Immobilisierung kann beispielsweise in einer Mikrotiterplatte erfolgen, in der dann mit Hilfe einer Molekül-markierten spezifischen Sonde, die an die Amplifikationsprodukte hybridisiert, und eines Enzymgekoppelten (beispielsweise einer Peroxidase) Antikörpers gegen das Markermolekül der Sonde eine Farbreaktion erfolgt. Diese Farbreaktion läßt sich anschließend photometrisch erfassen.

Das Oligonukleotid für einen Nachweis durch die in vitro Hybridisierung trägt im allgemeinen die oben beschriebenen Markierungen, wobei in Verbindung mit der NASBA-Amplifizierungsmethode die Hybridisierungsprobe eine Elektrochemolumineszenzmarkierung, vorzugsweise eine Tris[2,2-bipyridin]ruthenium[II]-Komplexmarkierung tragen kann [siehe z. B. van Gemen et al. (1994), J. Virol. Methods, 49, 157-168].

Eine genaue und sensitive Quantifizierung der Amplifikationsprodukte kann vorteilhafterweise auch dadurch erreicht werden, daß eine bestimmte Menge von einer oder mehreren bekannten Nukleinsäuren (Standard-Nukleinsäuren) co-amplifiziert wird [siehe z. B. van Gemen et al. (1993), J. Virol. Methods, 43, 177-188]. Hierbei wird zu den unbekannten Mengen der zu untersuchenden Probe eine unterschiedliche, jedoch genau bekannte Menge der co-amplifizierenden Standard-Nukleinsäure bzw. -Nukleinsäuren beispielsweise in Form einer Verdünnungsreihe hinzugegeben und die Amplifikationsprodukte der Probe und einer oder mehrerer co-amplifizierender Standard-Nukleinsäuren in unabhängigen Ansätzen quantitativ bestimmt. Ein Vergleich der Meßergebnisse ergibt die Menge der zu bestimmenden für die katalytische Untereinheit der Telomerase kodierenden mRNA in der Probe.

Vorteilhafterweise wird die co-amplifizierende Standard-Nukleinsäure bzw. -Nukleinsäuren mit demselben Oligonukleotid-Primer amplifiziert wie die zu untersuchende Probe und die Amplifikationsprodukte haben auch im wesentlichen die gleiche Länge. Besonders bevorzugt haben die co-amplifizierenden Nukleinsäuren dieselbe Sequenz wie das Amplifikationsprodukt der zu bestimmenden Probe mit Ausnahme von ca. 20 Nukleinsäuren zwischen den Oligonukleotid-Primer-Bindungsstellen, die eine willkürliche, jedoch bekannte Sequenz tragen. Dadurch können das zu bestimmende Amplifikationsprodukt in der Probe von dem co-amplifizierenden. Amplifikationsprodukt beispielsweise über eine Hybridisierung, wie z. B. bei Sambrook et al. (supra) näher beschrieben, mit entsprechend komplementären markierten Oligonukleotiden unabhängig voneinander quantifiziert werden. Insbesondere ist es vorteilhaft, wenn mehrere, vorzugsweise drei, verschiedene co-amplifizierende Nukleinsäuren in unterschiedlichen Konzentrationen der Probe zugesetzt werden, da hierdurch die Zahl der sonst einzeln durchzuführenden Amplifizierungsreaktionen reduziert werden kann [siehe van Gemen et al. (1994) J. Virol. Methods 49, 157-168]. Es ist auch insbesondere bevorzugt, die co-amplifizierenden Nukleinsäuren bereits zu dem oben beschriebenen RNA-Lysispuffer hinzuzugeben, da hierdurch der Einfluß möglicher Nukleinsäureverluste bei der nachfolgenden Aufarbeitung der Probe ausgeschlossen werden kann.

Als co-amplifizierende Standard-Nukleinsäuren gemäß der vorliegenden Erfindung eignen sich vorteilhafterweise RNA-Einzelstrang-Sequenzen, die durch in vitro Transkription beispielsweise mit der Sp6 oder T7 RNA Polymerase von Konstrukten hergestellt werden, die DNA oder cDNA der zu amplifizierenden Probe enthalten und die jeweils mit einem randomisierten Sequenzaustausch von beispielsweise ca. 10 bis ca. 30, vorzugsweise ca. 20 Nukleotiden ausgestattet sind.

Die Konstrukte bestehen bevorzugterweise aus einem Transkriptionsvektor mit einer Bindungsstelle für die Sp6 oder T7 RNA Polymerase zwischen einer sogenannten "Multiple Cloning Site", in welcher die DNA oder cDNA der zu amplifizierenden Probe kloniert worden ist. Durch eine selektive Hydrolyse mit Restriktionsendonukleasen, vorzugsweise mit zwei verschiedenen Restriktionsendonukleasen, kann die klonierte Sequenz geöffnet und ein Stück einer bestimmten Länge herausgeschnitten und durch ein gleich langes Stück beispielsweise mit Hilfe der T4 Ligase ersetzt werden. Das klonierte Stück kann einen Sequenzaustausch beliebiger Länge, beispielsweise ca. 10 bis ca. 30, vorzugsweise ca. 20 Nukleinsäuren enthalten und liegt vorzugsweise zwischen den Oligonukleotid-Primer-Bindungsstellen. Diesen Vorgang kann man wiederholen, um an gleicher Stelle Insertionen mit anderen Nukleinsäure-Sequenzen vorzunehmen. Lassen sich keine geeigneten Schnittstellen finden, z. B. weil auch im Vektor geschnitten wird, müssen künstliche Schnittstellen geschaffen werden. Dies kann beispielsweise mittels rekombinanter PCR geschehen, die im wesentlichen bei Higuchi et al. [Higuchi, R. (1988). Nucleic Acid Res 16: 7351-7367; Higuchi, R. (1990). M. Innis A. et al. eds. San Diego, New York, Berkley, Boston, London, Sydney, Tokyo, Toronto, Academic Press, Inc. 177-183] und im experimentellen Teil der vorliegenden Erfindung beschrieben ist.

Eine bevorzugte Verwendung im Rahmen der Erfindung finden in vitro transkribierte RNA-Einzelstrang-Sequenzen von Konstrukten, welche

a) die gesamte cDNA entsprechend der für die katalytische Untereinheit der menschlichen Telomerase kodierenden mRNA enthalten und

b) in welchen ein randomisierter Sequenzaustausch von ca. 20 Nukleotiden eingeführt worden ist.

Da sich die Standard-Nukleinsäuren (beispielsweise hTRTKa, hTRTKb und hTRTKc) untereinander und von der Wildtyp-Sequenz beispielsweise nur durch eine randomisierte und bekannte 20 Basenpaar lange Sequenz unterscheiden, können die Amplifikationsprodukte der Standard-Nukleinsäuren und der Wildtyp-Sequenz durch komplementäre Bindung von markierten Oligonukleotiden in vier getrennten Ansätzen nachgewiesen werden.

Zum Nachweis der amplifizierten "antisense" RNA, werden die entsprechenden reverse komplementären Sequenzen verwendet.

Danach werden die individuellen Amplifikationsmengen von dem Wildtyp- und den Standard-Nukleinsäuren bestimmt. Im Vergleich zu den unterschiedlichen Amplifikationsmengen der Standard-Nukleinsäuren bei bekannter Ausgangsmenge (z. B. hTRTKa: $10^2$, hTRTKb: $10^4$ und hTRTKc: $10^6$ Moleküle) läßt sich die unbekannte Ausgangsmenge der Wildtypsequenz errechnen. Daraus läßt sich dann auf die Anzahl der Metastasen pro abgenommener Körperflüssigkeit schließen.

Als interne positive Kontrolle des Verfahrens und der zu untersuchenden Probe kann zusätzlich eine Nukleinsäure amplifiziert und nachgewiesen werden, die im allgemeinen immer in einer Körperflüssigkeit vorkommt. Als geeignete Nukleinsäuren sind beispielsweise die mRNA kodierend für β-Globin oder für die Glycerinaldehydphosphatdehydrogenase (GAPDH) zu nennen (siehe z. B. GB 2 260 811), die immer in den Zellen der Körperflüssigkeit vorkommen. Geeignete Oligonukleotid-Primer für die humane ß-Globin mRNA sind beispielsweise Primer mit den Sequenzen:

5' ACCCAGAGGT TCTTTGAGTC 3' (Glob 1) und
5' TCTGATAGGC AGCCTGCACT 3' (Glob 2).

Weitere interne positive Kontrollen des Verfahrens und der zu untersuchenden Probe können zusätzlich zellspezifische Nukleinsäuren, wie ß-Aktin mRNA, mit den Primern (Nakajima-Iiji, S., Hamada, H., Reddy, P., Kakanuga, T. (1985): Molecular structure of the cytoplasmatic ß-actin gene: Interspezies homology of sequences in the introns. Proc Natl Acad Sci USA 82, 6133-7):

5’ GATGATGATATCGCCGCGCTCGTC 3’ (Act 1)
5’ CTCAAACATGATCTGGGTCATCTTC 3’ (Act 2)

oder T-Zell spezifische Nukleinsäuren, wie die mRNA des T-Zell-Rezeptors, mit den Primern (Toyonaga, B., Yoshikai, Y., Vadasz, V., Chin, B., Mak, T.W. (1985): Organization and sequences of the diversity, joining, and constant region of the human T-cell receptor β chain. Proc Natl Acad Sci USA 82, 8624-8):

5’ GAGGTCGCTGTGTTTGAGCCATCAGAAG 3’ (TCR 1)
5’ GATCTCATAGAGGATGGTGGCAGACAG 3’ (TCR 2)

sein.

Die Oligonukleotid-Primer können gegebenenfalls zusätzlich eine Promotorsequenz für eine RNA-Polymerase enthalten.

Zur Vermeidung bzw. Reduzierung falsch positiver Ergebnisse bzw. des sogenannten Hintergrundrauschens, das durch eventuell vorhandene Telomeraseaktivitäten von Nicht-Tumorzellen verursacht wird, wird die zu untersuchende Probe erfindungsgemäß zunächst einem Verfahren zur An- oder Abreicherung von Tumorzellen unterzogen. Hierzu ist es vorteilhaft, die entnommene Körperflüssigkeit in entsprechenden Verfahren entweder aufzureinigen oder zu Konditionen zu kultivieren, die für die kontaminierenden Telomerase-positiven Nicht-Tumorzellen ungünstig, jedoch für die vorhandenen Tumorzellen günstig sind.

Im Falle der Aufreinigung sollen insbesondere aus der zu untersuchenden Probe Stammzellen und/oder aktivierte Immunzellen abgereichert oder Tumorzellen angereichert werden. Da in der Regel die einzelnen Zellen spezifische Oberflächenmarker besitzen ist eine Abtrennung oder Anreicherung der Zellen über die Immunabsorption besonders vorteilhaft. Als Methoden eignen sich beispielsweise die magnetische (MACS) oder die fluoreszenzaktivierte (FACS) Zellsortierung [siehe z. B. Göttlinger & Radbruch (1993) mta, 8, 530-536, No. 5]. So können hämatopoetische Stammzellen beispielsweise über ihren Oberflächenmarker CD34 mittels MACS von der Blutprobe abgetrennt werden [Kato & Radbruch (1993) Cytometry, 14, 384-392]. B-Zellen lassen sich beispielsweise über ihre Oberflächenmarker CD10, CD19 und/oder CD20 und T-Zellen über CD45RA und/oder CD7 abtrennen. Tumorzellen können über ihre spezifischen Tumormarker, z. B. CEA, angereichert werden. Neben MACS oder FACS eignen sich auch besonders an sogenannte käuflich erhältliche Magnetbeads (z. B. Dynabeads M450, Dynal Corp.) gebundene Antikörper gegen die spezifischen Oberflächenmarker zur Abreicherung oder Anreicherung der entsprechenden Zellen.

Die Isolierung mononukleärer Zellen kann nach folgenden Methoden erfolgen:

spezifische Lyse der Erythrozyten (Bsp. Ammoniumchlorid/hypertonischer Schock),
spezifische Lyse mononukleärer Blutzellen (Bsp. L-Leucyl-L-Leucine-Methylester für Monozyten und zytotoxische Zellen), negativ/positiv Selektion mittels spezifischer Oberflächenmarker (Bsp. negativ: CD 4, 8, 34 etc.; positiv: Ber-EP4, AUA1, CEA),
Dichtegradienten-Zentrifugation euploider vs. aneuploider Zellen oder
Dichtegradienten-Zentrifugation von mononukleären kernlosen Zellen (Bsp. Ficoll).

In den 60er- und 70er-Jahren, als Methoden wie beispielsweise FACS oder MACS noch nicht zur Verfügung standen, wurden Tumorzellen von hämatopoetischen Zellen aufgrund ihrer unterschiedlichen Dichte getrennt (J.A. Fleming et al., J. clin. Path. (1967), 20, 145). Entsprechend dieser Daten besitzen Tumorzellen eine spezifische Dichte von 1,040-1,080, während Erythrozyten und polymorphnukleäre Leukozyten eine höhere Dichte aufweisen. Lymphozyten weisen hingegen eine spezifische Dichte im Bereich von 1,060-1,075 auf und überschneiden sich somit mit der spezifischen Dichte von Tumorzellen. Eine vollständige Abtrennung der ebenfalls Telomerase-aktiven Lymphozyten von den Tumorzellen über deren Dichteunterschiede sollte somit nicht möglich sein. So zeigte auch die Verwendung einer Standardlösung zur Isolierung von Lymphozyten, wie z.B. Histoprep® mit einer Dichte von 1,077 g/ml, daß Lymphozyten von gesunden Blutspendern mit einer Dichte von bis zu 1,077 g/ml eine Telomerase-Aktivität aufweisen.

Bei dem erfindungsgemäßen Verfahren erfolgt die Anreicherung der Tumorzellen dadurch, daß man ein Zellseparationsmedium mit einer Dichte im Bereich von 1,060 bis < 1,065 g/ml mit der Körperflüssigkeit, die die Tumorzellen enthält, überschichtet und zentrifugiert. Durch die Verwendung des speziellen Zellseparationsmediums werden die in der Körperflüssigkeit vorhandenen Zellen so aufgetrennt, daß die aufgrund ihrer Dichte zusammen mit den Tumorzellen angereicherten Lymphozyten keine Telomerase-Aktivität aufweisen.

Das Anreicherungsverfahren verwendet ein Zellseparationsmedium als diskontinuierlichen Gradienten, das mit der Körperflüssigkeit überschichtet wird. Durch Zentrifugation werden die Zellen ihrer spezifischen Zelldichte nach aufgetrennt und können in einzelnen Fraktionen abgenommen werden. Der spezifische Dichtegrad des Zellseparationsmediums erlaubt eine fast vollkommene Trennung von Tumorzellen von den in den Körperflüssigkeiten befindlichen korpuskulären Anteilen, speziell den Zellen des roten und weißen Blutsystems. Darüber hinaus erlaubt es das Verfahren, Telomerase-positive von Telomerase-negativen hämatopoetischen Zellen zu trennen, wobei sich die angereicherten Tumorzellen nach der Zentrifugation in der gleichen Fraktion befinden wie die Telomerase-negativen hämatopoetischen Zellen, so daß eine anschließend nachgewiesene Telomerase-Expression in dieser Fraktion zweifelsfrei auf vorhandene Tumorzellen zurückgeführt werden kann.

Überraschend ist auch, daß durch die gegenüber dem Stand der Technik nur geringfügige Verringerung der Dichte des Zellseparationsmediums eine erhebliche Reduzierung der kontaminierenden Blutzellen erreicht wird. Dadurch wird die Gesamt-Zellzahl signifikant verringert, ohne daß signifikante Verluste an Tumorzellen auftreten, wodurch z.B. das Screening von mikroskopischen Präparaten erheblich vereinfacht und im klinischen Maßstab erst möglich wird.

Es wurde gefunden, daß eine besonders gute Trennleistung mit einem Zellseparationsmedium einer Dichte im Bereich von 1,060-1,067 g/ml, bevorzugter von 1,060-1,065 g/ml und besonders bevorzugt von etwa 1,065 g/ml erzielt wird.

Die Zentrifugation wird vorteilhaft bei ca. 1.000 x g über ca. 30 Minuten durchgeführt. Die Temperatur während der Zentrifugation beträgt vorzugsweise ca. 4°C. Insbesondere hat es sich als vorteilhaft erwiesen, wenn die Zentrifugation mit langsamer Beschleunigung und ohne Bremsen durchgeführt wird, damit das Zellseparationsmedium und die Körperflüssigkeit zu Beginn der Zentrifugation bzw. die Fraktionen am Ende der Zentrifugation nicht miteinander vermischt werden.

Als Zellseparationsmedium läßt sich im Prinzip jede geeignete Flüssigkeit gewünschter Dichte verwenden. Das Zellseparationsmedium sollte nicht mit der Körperflüssigkeit oder den darin enthaltenen Zellen reagieren. Vorteilhaft kann beispielsweise Ficoll oder Percoll verwendet werden, wobei die Lösungen jeweils nach Anweisungen des Herstellers auf die gewünschte Dichte gebracht werden. Beispielsweise berechnet sich die Menge der zu verdünnenden Percoll-Stammlösung mit einer Dichte von 1,13 g/ml, die zur Herstellung von 100 ml einer Percoll-Arbeitslösung gewünschter Dichte (dd) benötigt wird, nach der Formel:

$$100\ \text{ml} \times (dd - 0{,}106 - 0{,}9)/0{,}13.$$

10% der Percoll-Arbeitslösung gewünschter Dichte bestehen immer aus einer 1,5 M NaCl-Lösung, um eine physiologische Osmolarität zu gewährleisten. Die Differenz zwischen der nach der vorstehenden Formel berechneten Menge an Percoll-Stammlösung (Dichte 1,13 g/ml) und der Salzlösung zu 100 ml wird dann mit Wasser aufgefüllt.

Damit läßt sich eine Percoll-Arbeitslösung mit einer Dichte von 1,060 g/ml beispielsweise wie folgt herstellen:

| | |
|---|---|
| 41,54 ml | der Percoll-Stammlösung (Dichte von 1,13 g/ml) |
| 48,46 ml | $H_2O$ |
| 10,00 ml | 1,5 M NaCl |
| 100,00 ml | Percoll-Arbeitslösung, dd 1,060 g/ml |

Die angegebenen Dichten beziehen sich auf eine Temperatur von 20°C. Vorteilhaft werden die Arbeitslösungen bei Raumtemperatur hergestellt und beispielsweise mit Hilfe von Marker-Beads einer definierten Dichte überprüft.

Wenn peripheres Blut als Körperflüssigkeit eingesetzt wird, wird dieses vorteilhaft in einer gerinnungshemmenden Substanz abgenommen und vor dem Überschichten des Zellseparationsmediums mit einem Verdünnungsmittel verdünnt. Als gerinnungshemmende Substanzen werden bevorzugt EDTA oder Citrat eingesetzt, als Verdünnungsmedium eignet sich beispielsweise PBS. Das Blut wird vorzugsweise im Verhältnis von ca. 1:1 verdünnt. Als peripheres Blut eignet sich venöses oder arterielles Blut.

Die zu untersuchende Körperflüssigkeit wird für die Anreicherung der Tumorzellen entsprechend gängiger Standardprotokolle entnommen bzw. gesammelt. In Abhängigkeit von der Art der Körperflüssigkeit wird diese dann entweder zunächst mit einem Verdünnungsmittel, bevorzugt einem Puffer, verdünnt oder direkt unverdünnt in einem Zentrifugationsgefäß über das Zellseparationsmedium geschichtet. Alternativ kann die Körperflüssigkeit zuvor bei beispielsweise 1.000 x g für ca. 10 Min. zentrifugiert und nach Resuspension der Zellen in einem Puffer über das Zellseparationsmedium geschichtet werden. Der bevorzugt verwendete Puffer ist Dulbecco PBS. Als Zentrifugationsgefäß eignet sich insbesondere ein silikonisiertes Zentrifugationsgefäß bevorzugt aus Kunststoff mit einer Kapazität von beispielsweise 1-500 ml. Das Zentrifugationsgefäß sollte verschließbar sein.

In einer weiteren bevorzugten Ausführungsform des Anreicherungsverfahrens werden der Körperflüssigkeit vor dem Überschichten eine oder mehrere Substanzen zugegeben, die eine Aggregation von Thrombozyten an Tumorzellen verhindern. Diese Substanzen können zum Beispiel mit dem als Verdünnungsmittel verwendeten Puffer zugesetzt werden. Als Substanzen, die eine unerwünschte Aggregation von Thrombozyten an Tumorzellen verhindern, eignen sich beispielsweise EDTA, Citrat und ACD-A (acid citrate dextrose). Zusätzlich oder statt dessen kann die Körperflüssigkeit vor dem Überschichten von Substanzen befreit werden, die eine Aggregation von Thrombozyten an Tumorzellen fördern. Hierbei handelt es sich beispielsweise um Ionen wie Magnesium- und Calciumionen.

Im Zentrifugationsgefäß wird das Zellseparationsmedium, das eine höhere Dichte als die zu untersuchende Körperflüssigkeit aufweist, vorgelegt, und anschließend mit der Körperflüssigkeit überschichtet. Je nach Größe des Zentrifugationsgefäßes und dem Volumen der Körperflüssigkeit, aus der die Tumorzellen angereichert werden sollen, kann das Zellseparationsmedium beispielsweise mit einem Volumen von 1-250 ml vorgelegt werden.

Als besonders vorteilhaft hat es sich erwiesen, wenn das untere Viertel des Zentrifugationsgefäßes nach der Zentrifugation und vor der Abnahme der an Tumorzellen angereicherten Interphase kurzzeitig stark abgekühlt wird um Zell-Kontaminationen zu verhindern. Beispielsweise können die im Zellpellet befindlichen Erythrozyten und Leucozyten dadurch immobilisiert werden, daß das untere Viertel des Zentrifugationsgefäßes für 5-10 Minuten in flüssigem Stickstoff stark abgekühlt wird. Als Interphase wird vorliegend der Übergang zwischen dem Zellseparationsmedium und der darüberliegenden Körperflüssigkeit bezeichnet. In dieser Interphase reichern sich die Tumorzellen an und werden nach der Zentrifugation beispielsweise durch Absaugen dieser Phase gesammelt. Durch das starke Abkühlen des Zentrifugationsgefäßes wird ein Vermischen der Zellen aus den verschiedenen Phasen verhindert, wodurch falsch-positive Testergebnisse ausgeschlossen werden können.

Um einen möglichst einfachen Ablauf der Arbeiten zu sichern, kann die Zentrifugation in einem Gefäß durchgeführt werden, das durch eine poröse Barriere, einen Filter oder ein Sieb, nachfolgend poröse Barriere oder Barriere genannt, in ein oberes und ein unteres Kompartiment geteilt ist, wobei das Zellsuspensionsmedium im unteren Kompartiment vorgelegt und die Körperflüssigkeit in das obere Kompartiment eingebracht wird. Hierdurch wird ein Vermischen der zu untersuchenden Körperflüssigkeit im oberen Kompartiment mit dem Zellseparationsmedium im unteren Kompartiment vor und nach dem Zentrifugationsschritt vermieden.

Die Position der porösen Barriere in dem Zentrifugationsgefäß kann dabei so gewählt werden, daß der Flüssigkeitsspiegel des Zellseparationsmediums entweder genau unterhalb, genau innerhalb oder genau oberhalb der porösen Barriere zu liegen kommt.

Die poröse Barriere kann beispielsweise eine Dicke von 1-10 mm, vorzugsweise ca. 5 mm aufweisen. Die poröse Barriere sollte darüber hinaus eine Festigkeit aufweisen, die es ihr erlaubt den Zentrifugationskräften unbeschadet Stand zu halten.

Eine bevorzugte Verwendung finden Barrieren mit einer porösen Beschaffenheit, die es erlaubt, daß bei der Zentrifugation Flüssigkeit sowie die korpuskulären Bestandteile des Blutes, wie die Zellen des roten und weißen Blutsystems, nicht aber die Tumorzellen die poröse Barriere ungehindert passieren können. Dies hat zur Folge, daß das Zellseparationsmedium während der Zentrifugation durch die poröse Membran in das obere Kompartiment gedrängt wird und die Tumorzellen und Thrombozyten auf einer Ebene oberhalb der Barriere zu liegen kommen. Hierzu eignen sich insbesondere poröse Barrieren mit einer Porengröße von 20-100 $\mu$m, vorzugsweise 20-30 $\mu$m.

Die poröse Barriere kann aus jedem geeigneten Material bestehen. Beispielsweise eignen sich Kunststoff, Metall, Keramik oder eine Mischung oder spezielle Legierung dieser Stoffe. Es kann aber auch jedes andere natürliche oder künstliche, geeignete Material eingesetzt werden.

In einer weiteren bevorzugten Ausführungsform besteht die poröse Barriere aus einem hydrophoben Material oder ist mit einem hydrophoben Material beschichtet.

Mit Hilfe der porösen Barriere kann die zu untersuchende Körperflüssigkeit in das Zentrifugationsgefäß gefüllt werden, ohne daß sie sich mit dem darunterliegenden Zellseparationsmedium vermischt und somit die Anreicherung beeinträchtigen oder unmöglich machen kann.

Nach der Zentrifugation befinden sich die in der Körperflüssigkeit vorhandenen Tumorzellen in der Interphase des oberen Kompartiments des Zentrifugationsgefäßes. Die Flüssigkeit oberhalb der Interphase kann dann zu etwa 80% vorsichtig abgesaugt und verworfen werden. Bei dieser Restflüssigkeit handelt es sich beispielsweise bei der Verwendung von Blut als Körperflüssigkeit um ein Plasma/PBS-Gemisch, das die Serumproteine enthält.

Der verbleibende Überstand oberhalb der Barriere, in dem sich die Tumorzellen befinden, kann anschließend gesammelt und beispielsweise in ein frisches Zentrifugationsgefäß (vorzugsweise aus silikonisiertem Kunststoff und mit der gleichen Volumenkapazität wie das zuvor verwendete Zentrifugationsgefäß) überführt werden. Die poröse Barriere verhindert bei der Entnahme des verbleibenden Überstands ein Vermischen der Zellen des oberen und des unteren Kompartiments.

Vorteilhaft wird anschließend das obere Kompartiment des Zentrifugationsgefäßes beispielsweise zweimal mit einem Puffer nachgewaschen und dieser wird ebenfalls in das frische Zentrifugationsgefäß überführt. Als Puffer eignen sich beispielsweise Dulbeccos PBS (3,9 mM EDTA, pH 8,0 ohne Calcium und Magnesium) oder NaCl/10% ACD-A (Guidlines for the collection, processing and storage of human bone marrow and peripheral stem cells for transplantation, prepared by the BCSH Blood Transufsion Task. Transfus. Med. 1994; 4: 165-72) oder NaCl/5% ACD-A/1% Albumin). Nach einer weiteren Zentrifugation beispielsweise bei 1.000 x g über ca. 10 Min. bei einer Temperatur von ca. 4°C können die gesammelten Zellen beispielsweise den Tumorzellnachweismethoden zugeführt werden.

Um den nach der Zentrifugation die Tumorzellen enthaltenden Zellring an der Interphase zwischen Zellseparationsmedium und Körperflüssigkeit für die Entnahme aus dem Zentrifugationsgefäß besser sichtbar zu machen, hat es sich als vorteilhaft erwiesen, dem Zellseparationsmedium einen Farbstoff zuzugeben. Beispielsweise können zu 100 ml einer Percoll-Arbeitslösung 80 μl einer 5% Trypan Blau-Lösung zugegeben werden.

Bei Verwendung eines Zentrifugationsgefäßes mit poröser Barriere wird nach Entnahme der überstehenden Restflüssigkeit oberhalb der Interphase jedoch bevorzugt nicht nur die Interphase sondern die gesamte verbliebene Flüssigkeit oberhalb der porösen Barriere entnommen, nicht weil sich zwischen Interphase und poröser Barriere noch weitere Zellen befinden, sondern weil durch die Abnahme die im Zellring enthaltenen Zellen leicht durchmischt werden können. Um keine Zellen aus dem oberen Kompartiment zu verlieren wird dieses vorteilhaft noch zweimal mit Puffer (z.B. PBS) gewaschen.

Mit dem Anreicherungsschritt können zirkulierende Tumorzellen und insbesondere zirkulierende Tumorzellen von soliden Tumoren, d.h. von nicht-hämatologischen Tumoren, angereichert werden oder hämatologische Tumorzellen, d.h. Tumorzellen des roten und weißen Blutsystems.

Mit dem Anreicherungsverfahren können Tumorzellen aufgrund ihrer unterschiedlichen Dichte nahezu vollständig von den korpuskulären Bestandteilen von Körperflüssigkeiten wie z.B. den Zellen des roten und weißen Blutsystems getrennt, konzentriert und anschließend dem nächsten Schritt des erfindungsgemäßen Quantifizierungsverfahrens zugeführt werden.

Das beschriebene Anreicherungsverfahren bietet den Vorteil, daß Telomerase-positive hämatopoetische Zellen einfach und sicher von den anzureichernden Tumorzellen abgetrennt werden, so daß im anschließenden Quantifizierungsschritt keine falsch-positiven Ergebnisse durch Telomerase-aktive nicht-Tumorzellen erhalten werden. Darüber hinaus sind nur wenige Arbeitsschritte für die Anreicherung und Isolierung der Tumorzellen aus Körpergewebe notwendig, so daß dadurch die Prozessierung von größeren Mengen von Probenmaterial möglich wird. Die Kosten für die notwendigen Materialien sind beispielsweise gegenüber der Verwendung spezifischer Antikörper und der anschließenden Trennung mittels geeigneter Apparaturen signifikant geringer.

Darüber hinaus zeigte die Untersuchung von 10 unterschiedlichen Zellinien, die von Tumorgeweben, wie Melanom, Prostata-, Brust-, Lungen-, Leber- und Colorektalkarzinomen abgeleitet waren, daß die Zellen all dieser Zellinien in ihrer Mehrzahl durch das beschriebene Anreicherungsverfahren angereichert wurden.

In einer alternativen Vorgehensweise zur Auftrennung der Blutzellen in Ficoll oder Percoll können die in der Probe enthaltenen Zellen, unter Konditionen kultiviert werden, die für kontaminierende Telomerase-positive Nicht-Tumorzellen ungünstig, jedoch für die vorhandenen Tumorzellen günstig sind. Das Resultat dieser Kultivierungsverfahren ist, daß kontaminierende Telomerase-positive Nicht-Tumorzellen absterben während vorhandene Tumorzellen überleben.

Hierfür kann zur Optimierung der Zellkultur, um die Proliferation der Tumorzellen einerseits und den Eintritt der Blutzellen in die $G_0/G_1$-Phase bzw. deren Apoptose anderseits zu favorisieren, um vorhandene metastasierende Tumorzellen anzureichern, die Probe folgenden Bedingungen ausgesetzt werden:

Auswahl geeigneter Kulturmedien unter Verwendung (autologer) Seren,
Dauer der Kultivierung,
Auswahl des optimalen Sauerstoff-/Kohlendioxidgehalts, Zugabe von Tumor-Zell-/Epithelzell-spezifischen Wachstumsfaktoren (Bsp. EGF etc.) oder
Auswahl einer geeigneten Oberflächen-Beschichtung, um die Adhärenz der Tumorzellen in Zellkulturbehältern zu erhöhen und Selektion von adhärenten Zellen gegenüber Suspensionszellen.

Die Kultivierung kann somit beispielsweise wie folgt ausgeführt werden:

Die in der Probe enthaltenen Zellen können gesamt oder in Aliquoten (z.B. auf Mikrotiterplatten) mit oder ohne Zusätze in Kultur genommen werden. Diese Kulturen können in Folge Einflüssen ausgesetzt werden, die Nicht-Tumorzellen absterben, jedoch Tumorzellen überleben lassen. Die Einflüsse können beispielsweise allein schon die Dauer der Kultivierung, interne (z.B. Anstieg oder Abnahme des pH-Wertes im Kulturmedium) oder externe Einflüsse (wie z.B. Erhöhung oder Erniedrigung von $pCO_2$ oder $pO_2$) sein. Unsere Untersuchungen haben z.B. gezeigt, daß schon bei einer einfachen Kultivierung von Vollblut bei 37°C für 5 Tage, die mRNA kodierend für die der katalytische Untereinheit der menschlichen Telomerase (hTRT) zwischen Tag 1 und Tag 2 nicht mehr nachweisbar war. Noch nachzuweisen waren hingegen die mRNA für den T-Zell-Rezeptor (TCR) (3 Tage) und ß-Aktin (5 Tage) (vgl. Abb. 4).

Die Kultivierung der in den Flüssigkeiten enthaltenen Nicht-Tumorzellen und Tumorzellen nach den oben genannten Verfahren kann auch zusätzlich sowohl vor als auch nach Separation der Zellen aus der Körperflüssigkeit (z.B. nach Gradientenzentrifugation z.B. mit Fiquoll-Hypaque) erfolgen.

Allein oder in Verbindung mit den oben beschriebenen Reinigungs- bzw. Kultivierungsverfahren ist es auch besonders vorteilhaft die Menge an für die katalytische Untereinheit der Telomerase kodierender mRNA aus venösem Blut mit der Menge an für die katalytische Untereinheit der Telomerase kodierender mRNA aus arteriellem Blut zu vergleichen, da bei venöser Blutabnahme zum Zwecke der Tumorzellbestimmung etwa nur 20% aller Zellen gegenüber 100% der Zellen bei arterieller Blutabnahme nachweisbar sind [Koop, S. et al. (1995) Cancer Res. 55, 2520-2523]. Ebenso eignet sich ein Vergleich von Blut aus der Fingerkuppe mit venösem oder arteriellem Blut.

Die quantitative Bestimmung der für die katalytische Untereinheit der Telomerase kodierenden mRNA in der Probe ermöglicht es, zu bestimmen, ob Tumorzellen, insbesondere Metastasen, vor allem Mikrometastasen, von malignen Tumoren in der Körperflüssigkeit enthalten sind und in welcher Menge. Dies ist insbesondere für die frühzeitige klinische Diagnose der Metastasenbildung von malignen Tumoren und für die Überwachung einer Tumortherapie von großem Nutzen. Als Tumorzellen können mit der vorliegenden Erfindung insbesondere Tumorzellen von Metastasen, vorzugsweise Mikrometastasen, von malignen Tumoren, vor allem Zellen metastasierender Tumore und/oder Neoplasien nachgewiesen werden, die beispielsweise von einem T-Zell-Lymphoblastom, T-Zell-Leukämiezellen, chronisch myeloische Leukämiezellen, akute lymphatische Leukämiezellen, chronisch lymphatische Leukämiezellen, Teratokarzinom, Melanom, Lungenkarzinom, Dickdarmkrebs, Brustkrebs, Leberzellkarzinom, Nierentumor, Nebennierentumor, Prostatakarzinom, Neuroblastom, Gehirntumor, kleines Lungenzellkarzinom, Rhabdomyosarkom, Leiomyosarkom und/oder Lymphom abstammen.

Weitere Gegenstände der vorliegenden Erfindung sind die Oligonukleotid-Primer mit der Sequenz
5’ CTACCGGAAG AGTGTCTGGA GCAAGTTGCA AAGC 3’ (hTRT1) und/oder
5’ GGCATACCGA CGCACGCAGT ACGTGTTCTG 3’ (hTRT2),
wobei hTRT1 und/oder hTRT2 gegebenenfalls zusätzlich eine Promotorsequenz für eine RNA-Polymerase enthalten können;
sowie ein Oligonukleotid mit der Sequenz
5’ CGTTCTGGCT CCCACGACGT AGTC 3’ (hTRT o)
als Oligonukleotid-Sonde und die entsprechende reverse komplementäre Sequenz des Oligonukleotids zum Nachweis der amplifizierten "antisense" RNA.

Ein anderer Gegenstand der Erfindung ist ein Kit zur Quantifizierung von Tumorzellen in einer Körperflüssigkeit, beispielsweise Blut, Urin aber auch Stuhl, Exsudate oder Transudate von Körperhöhlen, insbesondere peripheres Blut, enthaltend

(a) ein Oligonukleotid-Primerpaar zur spezifischen Amplifizierung von Telomerase-kodierender Nukleinsäure, wobei das Oligonukleotid-Primerpaar vorzugsweise folgende Sequenzen aufweist:

5’ CTACCGGAAG AGTGTCTGGA GCAAGTTGCA AAGC 3’ (hTRT1) und/oder
5’ GGCATACCGA CGCACGCAGT ACGTGTTCTG 3’ (hTRT2),

wobei hTRT1 und/oder hTRT2 gegebenenfalls zusätzlich eine Promotorsequenz für eine RNA-Polymerase enthalten können.

Der erfindungsgemäße Kit umfaßt in einer weiteren Ausführungsform zusätzlich (b) eine Nukleinsäure bzw. Nukleinsäuren zur Co-Amplifikation, wobei vorzugsweise 3 RNA-Standard-Nukleinsäuren zur Co-Amplifikation enthalten sind.

Der erfindungsgemäße Kit kann auch zusätzlich, wie oben näher beschrieben, ein markiertes Oligonukleotid als Hybridisierungsprobe zum spezifischen Nachweis der amplifizierten cDNA der Wildtypsequenz und/oder mehrere markierte Oligonukleotide als Hybridisierungsprobe zum spezifischen Nachweis der amplifizierten cDNA der Standard-Nukleinsäure bzw. -Nukleinsäuren enthalten. Darüber hinaus kann ein erfindungsgemäßer Kit für die PCR-Amplifikation zusätzlich die oben näher beschriebenen Enzyme, gegebenenfalls markierte Nukleotide und/oder geeignete Puffer enthalten, wie z. B. eine reverse Transkriptase, vorzugsweise eine AMV reverse Transkriptase, eine DNA-Polymerase, vorzugsweise eine Taq-Polymerase und/oder eine DNase und gegebenenfalls zur Abreicherung von Stammzellen und/oder aktivierten Immunzellen und/oder zur Anreicherung von Tumorzellen geeignete Mittel, wie oben bereits näher beschrieben.

Ein anderer erfindungsgemäßer Kit für die NASBA-Amplifikation kann ebenso neben den oben näher beschriebenen Standard-Nukleinsäuren ein markiertes Oligonukleotid als Hybridisierungsprobe zum spezifischen Nachweis der amplifizierten "antisense" RNA der Wildtypsequenz und/oder mehrere markierte Oligonukleotide als Hybridisierungsprobe zum spezifischen Nachweis der amplifizierten "antisense" RNA der Standard-Nukleinsäure bzw. -Nukleinsäuren enthalten. Zusätzlich kann er ebenso die oben näher beschriebenen Enzyme, gegebenenfalls markierte Nukleotide und/oder geeignete Puffer, wie z. B. eine reverse Transkriptase, vorzugsweise eine AMV reverse Transkriptase, eine RNA-Polymerase, vorzugsweise eine T7 RNA-Polymerase, eine RNase H und/oder eine DNase enthalten, und gegebenenfalls zur Abreicherung von Stammzellen und/oder aktivierten Immunzellen und/oder zur Anreicherung von Tumorzellen geeignete Mittel, wie oben bereits näher beschrieben.

Die folgenden Beispiele und Figuren sollen die vorliegende Erfindung näher beschreiben, ohne sie jedoch darauf zu beschränken.

Beschreibung der Figuren

Abb. 1 zeigt die von Nakamura et al. beschriebene Sequenz, kodierend für die katalytische Untereinheit der menschlichen Telomerase, von 4015 Basenpaaren (bp) und die Position der entworfenen Oligonukleotid-Primer bzw. die Oligonukleotid-Sonde (hTRT o) (unterstrichen): 5'-Primer hTRT1 (Position 1780-1813), 3'-Primer hTRT2 (Position 2261-2290) mit einem Amplifikationsprodukt von 513 Basenpaaren (bp) und die Sonde hTRT o (Position 1964-1987).

Abb. 2 zeigt (A) eine schematische Darstellung der genomischen Organisation der menschlichen Telomerase (hTRT). Das Telomerase-Protein weist eine Telomerase-spezifische Region auf (T, schwarz markiert) und besitzt Homologien zu konservierten Regionen von Reversen Transkriptasen verschiedener Viren (1, 2 und A bis E, schraffiert). Das verwendete Primerpaar (hTRT1, hTRT2) ist durch Pfeile dargestellt und ist in einer Region lokalisiert, die keine Homologien mit viralen Reversen Transkriptasen aufweist. (B) zeigt eine Agarose-Gelelektrophorese einer RT-PCR-Amplifikation mit hTRT-spezifischen Primern an RNA der humanen Teratokarzinom-Zellinie Tera2. Bande M: Marker, Bande 1 RT-PCR-Amplifikation, Bande 2: entsprechende DNA-Kontrolle ohne Reverse Transkriptase Reaktion.

Abb. 3 zeigt eine Agarose-Gelelektrophorese (a) der RT-PCR-Produkte mit TCR-spezifischen Primern an RNA und DNA von humanen PBMC und der humanen Fibroblasten-Zellinie MRC5 und die entsprechende Southern-Blot-Analyse mit einer TCR-spezifischen Oligonukleotid-Sonde (TCR-Sonde). M: Marker.

Abb. 4 zeigt eine Agarose-Gelelektrophorese der RT-PCR-Produkte mit spezifischen Primern für hTRT (A) bzw. TCR und Aktin (B) an aus 1 ml Vollblut isolierter RNA, nach 0-5 Tagen Kultur sowie eine Reaktion an der RNA der humanen Teratokarzinom-Zellinie Tera2. Banden $0^d$-$5^d$: Zeitraum der Kultur von respektive 0 bis 5 Tagen. M: Marker, Tera2: humane Teratokarzinom-Zellinie Tera2.

Abb. 5 zeigt eine Agarose-Gelelektrophorese der RT-PCR-Produkte an RNA der Prostata-Karzinom Zellinie LNcap. Banden $10^3$-$10^{-2}$: Verwendete Zellzahl pro Ansatz. DNA: DNA Kontrolle. $H_20$: Wasser Kontrolle. M: Marker. Die PCR wurde, wie in Abb. 7 angegeben mit spezifischen Primern (Abb. 6) für die katalytische Untereinheit der humanen Telomerase (A) und β-Aktin, mit 40 Zyklen durchgeführt. Unter den beschriebenen RT-PCR-Bedingungen ist die für die katalytische Untereinheit der humanen Telomerase kodierende mRNA in bis zu 10 Zellen nachweisbar (Pfeil). Zum Ausschluß von DNA-Kontaminationen wurde RNA von $10^3$ Zellen ohne Reverse Transkriptase als Kontrollreaktion sowie eine Wasser-Kontrolle ($H_2O$ in der RT-PCR mitgeführt.

Abb. 6 zeigt die Sequenzen der vorliegend eingesetzten Oligonukleotide.

Abb. 7 zeigt ein Flußdiagramm für das erfindungsgemäße Verfahren.

Abb. 8 zeigt das Ergebnis einer RT-PCR-Analyse von Blut eines gesunden Spenders (A) und Blut des gleichen Spenders gemischt mit GFP-transfizierten Zellen der Melanom-Zellinie T289 (B, C) nach Tumorzellenanreicherung mit einem Zellseparationsmedium einer Dichte von 1,070 g/ml.

Abb. 9 zeigt das Ergebnis einer RT-PCR-Analyse von Blut gesunder Spender, das mit Tumorzellen einer Prostata-Karzinom- (A) und Mamma-Karzinom-Zellinie (B) gemischt wurde, nach Tumorzellanreicherung mit einem Zellseparationsmedium einer Dichte von 1,065 g/ml.

Abb. 10 zeigt die Wiederfindungsrate von GFP-transfizierten Melanomzellen, die zu Blut unterschiedlicher (A, B) gesunder Spender gemischt wurden, nach Anreicherung mit einem Zellseparationsmedium einer Dichte von 1,065 g/ml.

BEISPIELE

Wenn nicht anders vermerkt, wurden die folgenden Beispiele nach Standardverfahren, wie z. B. bei Sambrook, J. et al. (1989) supra beschrieben, oder nach den Herstellerangaben der verwendeten Kits bzw. Enzyme durchgeführt.

1. Tumorzellanreicherung

1.1 Allgemeines Vorgehen

In einem silikonisierten Kunststoff-Zentrifugationsgefäß wurde venöses Blut (5-20 ml), mit EDTA versetzt (3,9 mM Endkonzentration, pH 8,0) und mit 1 Volumen PBS gemischt. Das Blut/PBS-Gemisch wurde anschließend auf 5-10 ml Percoll mit einer Dichte von 1,065 g/ml gegeben und bei langsamer Beschleunigung und ohne Bremse für 30 min. bei 1.000 x g und 4°C zentrifugiert. Das untere Viertel des Zentrifugationsgefäßes wurde anschließend für 5-10 min in flüssigem Stickstoff inkubiert. Dadurch wurde während des Absaugens der Zellen, die sich an der Interphase im Übergang zwischen dem Percoll und dem darüberliegenden Plasma/PBS-Gemisch befanden, eine Kontamination mit Zellen des Pellets verhindert. Die Zellen der Interphase, bei denen es sich vorwiegend um Thrombozyten und um im Blut zirkulierende Tumorzellen handelte, wurden anschließend in ein neues silikonisiertes Kunststoff-Zentrifugationsgefäß übertragen und für 10 min bei 1.000 x g und 4°C zentrifugiert. Für die anschließende RT-PCR-Untersuchung wurde das Zellpellet in einem Guanidium-Isothiocyanat-Puffer aufgenommen, wodurch die Zellen lysiert wurden und einer RNA-Isolierung unterzogen werden konnten.

1.2 Spiking-Experiment

Mit Hilfe von sogenannten Spiking-Experimenten, bei denen Tumorzellen verschiedener Zellinien zum Blut normaler Spender gemischt wurden und die Tumorzellen anschließend re-isoliert und in der RT-PCR untersucht wurden, wurde gezeigt, daß in Abhängigkeit der verwendeten Zellinie die Telomerase-Aktivität von etwa 1-4 gespikter Tumorzellen/ml Blut nachgewiesen werden kann.

Hierzu wurden die Zellen der zu spikenden Tumorzellinien entsprechend der Angaben des Herstellers (ATCC, *American Tissue Cell Culture)* bis zur Konfluenz kultiviert. Die Zellen wurden anschließend trypsiniert und in Medium (RPMI 1640) gewaschen. Nach Entnahme eines 10 µl Aliquots, das 1:1 mit Tryptan-Blau gemischt wurde, wurden die lebenden Zellen in einer Zählkammer bestimmt und die entsprechende Zellkonzentration wurde berechnet. Anschließend wurde die Zellsuspension verdünnt und ein Volumen, das einer bestimmten Zellzahl entspricht, mit dem Blut gesunder Blutspender gemischt. Als Kontrolle diente Blut dem keine Tumorzellen zugesetzt wurden. Die Anreicherung der gespikten Tumorzellen wurde einmal zum Vergleich mit einem Zellseparationsmedium einer Dichte von 1,070 g/ml und nach dem erfindungsgemäßen Verfahren durchgeführt. Zur Bestimmung der Wiederfindungsrate wurden anschließend mikroskopische, durchflußzytometrische und RT-PCR-Analysen durchgeführt.

a) Vergleichsversuch

Abb. 8 zeigt das Ergebnis einer RT-PCR-Analyse von 20 ml Blut eines gesunden Spenders (A) und 20 ml Blut des gleichen Spenders gemischt mit GFP-transfizierten Zellen der Melanom-Zellinie T289 (B, C). Das Blut wurde auf Percoll einer Dichte von 1,070 g/ml geschichtet, zentrifugiert und anschließend wurden die Zellen analysiert. Die katalytische Untereinheit der Telomerase (hTRT) ist im normalen Blut nicht nachweisbar (A), während bei 1 und 2 gespikten Melanomzellen pro ml Blut hTRT nachweisbar ist (B, C). Bei der verwendeten Percoll-Dichte von 1,070 g/ml sind jedoch noch hinreichend viele Telomerase-aktive Leukozyten in der Interphase vorhanden, wodurch die RNA-Komponente (hTR) auch im ungespikten Blut nachweisbar ist. Für die Präsenz von aktivierten und wahrscheinlich deshalb auch Telomerase-aktiven Leukozyten in der Fraktion der isolierten Zellen spricht auch die Tatsache, daß CD69, ein früher Aktivierungsmarker in B- und T-Zellen, in allen Blutproben nachweisbar ist (A-C). Der Tumormarker CEA (Carcinoembrionic Antigene) ist sowohl im ungespikten als auch im gespikten Blut negativ (A-C). GFP (Green Fluorescent Protein), der als zusätzlicher Marker für die gespikten Tumorzellen verwendet wurde, ist im ungespikten Blut nicht nachweisbar (A). Da nur etwa 50% der transfizierten T289-Melanomzellen GFP exprimieren, ist das Protein nur in bis zu 2 gespikten Tumorzellen pro ml Blut nachweisbar (B). Aktin diente als RT-PCR-Positivkontrolle (Aktin) und im Ansatz ohne RT-Reaktion als Negativkontrolle (Aktin ØRT). Die PCR-Amplifikation genomischer DNA untransfizierter T289-Zellen führt mit den spezifischen Primerpaaren für hTRT, GFP und CD69 zu keinen Amplifikaten.

b) erfindungsgemäßer Versuch

Abb. 9 zeigt RT-PCR-Analysen von Blut gesunder Spender das mit Tumorzellen einer Prostata-Karzinom- (A) und Mamma-Karzinom-Zellinie (B) gemischt, auf Percoll einer Dichte von 1,065 g/ml geschichtet, zentrifugiert und anschließend analysiert wurde. Die RNA-Komponente der Telomerase (hTR) ist im Unterschied zu der Verwendung von Percoll mit einer Dichte von 1,070 g/ml im ungespikten Blut nicht nachweisbar (vgl. Fig. 1). In den Proben mit 2 gespikten Prostata-Karzinomzellen (A) bzw. mit 4 gespikten Mamma-Karzinomzellen (B) pro ml Blut kann hTR nachgewiesen werden (schwarzer Pfeil). Im Unterschied zur Melanom-Zellinie T289 konnte bei diesen Tumorzellen keine (A) bzw. erst bei $10^4$ Tumorzellen (B) eine Expression der katalytischen Untereinheit (hTRT) nachgewiesen werden. Weder der Prostatazell-spezifische Marker PSA (Prostate Specific Antigene) noch der Epithelzellspezifische Marker CK20 (Cytokeratin 20) ist in den entsprechenden Tumorzellen nachweisbar. Aktin dient als RT-PCR-Positivkontrolle.

Abb. 10 zeigt die Wiederfindungsraten von GFP-transfizierten Melanomzellen (T289), die zu Blutproben gesunder Spender gemischt (gespikt) wurden. Die gespikten Blutproben wurden anschließend auf Percoll einer Dichte von 1,065 g/ml geschichtet, zentrifugiert und die Anzahl der re-isolierten Tumorzellen (Wiederfindung) wurde mikroskopisch (-●-) und/oder durchflußzytometrisch (-▲-) bestimmt. Da nur etwa 75% für Probe A bzw. 50% für Probe B der GFP-transfizierten T289-Zellen im Durchflußzytometer nachweisbar waren, wurden die Wiederfindungsraten entsprechend korrigiert. Die Wiederfindungsrate gespikter Tumorzellen ist abhängig vom jeweiligen Blutspender (die Blutproben von A) und B) stammen von unterschiedlichen Spendern), der verwendeten Zellinie und verhält sich umgekehrt proportional zur Anzahl der gespikten Tumorzellen. Möglicherweise führt eine Abstoßungs-Reaktion der entsprechenden hämatopoetischen Zellen zur Lyse, Aggregation und schließlich zum Verlust der gespikten allogenen Tumorzellen. B) zeigt darüber hinaus, daß die Anzahl der tatsächlich gespikten Tumorzellen (-■-) zwischen 6% - 37% geringer ist als die theoretisch berechnete Anzahl gespikter Tumorzellen (-♦-).

Unter Hinzunahme hier nicht dargestellter Untersuchungen mit Lungen- und Mamma-Karzinomzellen ergibt sich eine durchschnittliche Wiederfindungsrate mit der erfindungsgemäß bevorzugten Anreicherungsmethode von 46% ± 20% für 4-512 gespikte Zellen (n=16) und für ≤ 50 gespikte Zellen von 54% ± 20% (n=15).

Damit liegt die Wiederfindungsrate des erfindungsgemäß bevorzugten Tumorzellenanreicherungsverfahrens etwa im Bereich von magnetischen Zell-Separatoren wie MACS für die eine Wiederfindungsrate von etwa 30-58% angegeben wird.

2. Kultivierung und Isolierung von peripherem Blut, Gewebe und Zellen für die nachfolgenden Beispiele

Tumorzellinien wie die menschliche Prostata-Karzinom-Zellinie LNcap und die Teratokarzinom Zell-Linie Tera 2 wurden gemäß den Vorgaben der ATCC (American Tissue Culture Collection), in Kultur genommen. Venöses Spenderblut von gesunden Kontrollpersonen wurde mittels Punktion einer Unterarmvene in EDTA-Monovetten® (Saarsted) abgenommen.

3. Isolierung von zellulärer RNA

Die Isolierung von totaler zellulärer RNA erfolgte nach Standardverfahren [Chomczynski et al. (1987) Anal Biochem 162, 156; Sambrook, J. et al. (1989). Cold Spring Harbor, New York, USA, Cold Spring Harbor Laboratory Press]. Peripheres Blut wurde wie in Abb. 7 gezeigt sofort nach Abnahme in RNA-Lysispuffer (4 M Guanidinium Isothiocyanat; 0.1 M Tris-HCl, pH 7.5; 1% Mercaptoethanol) überführt und homogenisiert. Die Gemische wurden entweder sofort weiterverarbeitet oder bei -70°C gelagert.

4. Reverse Transkription und Polymerase-Kettenreaktion (RT-PCR)

Die RT-PCR wurde mit dem GeneAmp® RNA-PCR-Kit (Perkin Elmer) nach Vorgaben des Herstellers wie in Abb. 7 gezeigt durchgeführt. Aliquote der isolierten totalen RNA von peripherem Blut und Zellinien wurden jeweils zuvor mit 4U DNAse und 40U RNAse Inhibitor (Boehringer, Mannheim) in 36 μl Ansätzen (in 100 mM Tris-HCl, pH 8.3; 50 mM KCl; 5 mM $MgCl_2$, 1mM dNTP-Mix und 2,5 mM Random Hexamers) bei 37°C für 30 Minuten und bei 75°C für 10 Minuten hydrolysiert und anschließend die DNAse für 10 Minuten bei 90°C inaktiviert und dann das Reaktionsgemisch sofort auf Eis gegeben.

Die beiden Oligonukleotid-Primer:

5' CTACCGGAAG AGTGTCTGGA GCAAGTTGCA AAGC 3' (hTRT1) und
5' GGCATACCGA CGCACGCAGT ACGTGTTCTG 3' (hTRT2)

wurden nach der von Nakamura et al. veröffentlichten Sequenz, kodierend für die katalytische Untereinheit der menschlichen Telomerase (Nakamura et al. (1997). Science 277: 955-9) entworfen (Abb. 1) und mit einem Applied Biosystem 380A Synthesizer synthetisiert. Die Spezifizität der hTRT1- und hTRT2-Primer wurde mittels Computer gestützter Homologieanalyse an den Nukleinsäuresequenzen in den GenBank-, EMBL-, DDBJ- und PDB-Datenbanken mittels BLASTN 1.4.9 MP [Altschul, S. F. et al. (1990). J Mol Biol 215: 403-410] überprüft.

Zur Abstimmung der Amplifikationsmengen wurden für jedes Experiment gleiche RNA-Mengen für die RT-Reaktion eingesetzt. Um eine Kontamination der RNA Präparationen mit genomischer DNA auszuschließen, wurde jede mit DNase hydrolysierte RNA-haltige Probe zuerst der unten beschriebenen PCR unterzogen und auf Amplifikation hin überprüft. Die RNA-haltige Probe, bei der kein Amplifikationsprodukt nachweisbar war, wurde für die folgenden cDNA-Synthese- und PCR-Schritte eingesetzt. Als interne Standardkontrolle wurden Oligonukleotid-Primer für β-Aktin und den TCR eingesetzt (Abb. 6). Die Reverse Transkriptase-Reaktion wurde an 18 μl des DNAse Verdaues unter Zugabe von 50U MuLV Reverser Transkriptase und 40U RNase Inhibitor bei 42°C für 30 Minuten durchgeführt und die Reaktion bei 99°C für 5 Minuten abgebrochen. In den Negativkontrollen wurden statt der Enzyme 4 μl Wasser zugesetzt.

Die PCR wurde wie in Abb. 7 gezeigt an 5 μl der cDNA-Reaktion nach folgendem Programm durchgeführt: (97°C: 15 Sekunden vorwärmen); (97°C: 15 Sekunden, 70°C: 30 Sekunden [minus 0.5°C pro Zyklus], 72°C: 30 Sekunden) 10 Zyklen; (94°C: 15 Sekunden, 65°C: 30 Sekunden [minus 0.5°C pro Zyklus), 72°C: 30 Sekunden) 20 Zyklen; (94°C: 15 Sekunden, 50°C: 30 Sekunden 72°C: 30 Sekunden [plus 15 Sekunden Extension pro Zyklus], 10 Zyklen; (72°C: 7 Minuten, finale Extension).

Die Amplifikationsprodukte wurden gelelektrophoretisch auf 1.5%igem TAE Agarosegel aufgetrennt, mit Ethidiumbromid gefärbt und unter UV-Licht visualisiert und fotodokumentiert (siehe Abb. 2-5).

5. Herstellung möglicher RNA-Standard-Nukleinsäuren (hTRTKa, hTRTKb und hTRTKc)

Die zur Klonierung bestimmten PCR-Amplifikationsprodukte können gelelektrophoretisch auf 1.5% TAE Agarose aufgetrennt und eluiert (Qiagen) werden. Die Aufreinigung der Restriktionshydrolysate kann durch Phenol-Chlorophorm-Extraktion und Fällung in Salz und Ethanol oder durch DNS-Reinigung (Qiagen) geschehen. Die Konstrukte können, z.B. durch Ligierung der Fragmente in die korrespondierenden Schnittstellen eines Klonierungs- und Transkriptionsvektors, z.B. pGEM-13zf(+), mit Hilfe der T4 Ligase geschaffen werden. Dieser Vektor erlaubt die in vitro Transkription von klonierten Fragmenten durch den wahlweisen Einsatz von Sp6- oder T7 RNA Polymerasen. Kompetente Bakterien (XL-1Blue, Stratagen) werden mittels Elektroporation (BioRad) transformiert. Plasmid DNA wird mittels Plasmid Reinigungs Kits (Qiagen) gereinigt. Positive Klone werden, unter Verwendung von vektor- oder sequenzspezifischen Oligonukleotid-Primern, mit der PCR validiert. Eine Sequenzvalidierung der Konstrukte kann durch semiautomatische Sequenzanalyse erfolgen.

Das Konstrukt pGEM-hTRT wird als Ausgangskonstrukt für die Konstrukte pGEM-hTRT(Ka) pGEM-hTRT(Kb) und pGEM-hTRT(Kc) geschaffen. pGEM-hTRT(Ka), pGEM-hTRT(Kb) und pGEM-hTRT(Kc) unterscheiden sich von pGEM-hTRT und untereinander durch einen randomisierten Sequenzaustausch von ca. 20 Basen Paaren (bp). Die Konstrukte werden zur in vitro Transkription mit Sp6 RNA Polymerase der Standard-RNA: hTRT(Ka), hTRT(Kb) und hTRT(Kc), verwendet. Zur Bildung des Konstrukts pGEM-hTRT wird die cDNA der katalytischen Untereinheit der menschlichen Telomerase (Abb. 1) z.B. in die NotI und HindIII-Schnittstellen von pGEM-13zf(+) kloniert. Dies wird dadurch erreicht, daß mit diese Schnittstellen enthaltenen Oligonukleotid-Primern die aus der Sequenz hTRT (Abb. 1) abgeleitet werden, eine RT-PCR an der bereits gewonnenen RNA aus Tumorzellen oder -linien unter den oben beschriebenen Bedingungen durchgeführt wird. Damit kann z.B. die mit vorgegebenen Schnittstellen versehene Voll-Längen hTRT oder ein kürzeres Fragment amplifiziert werden. Nach einer Restriktionshydrolyse mit spezifischen Restriktionsenzymen z.B. wie NotI und HindIII, wird das entstandene Fragment in die korrespondierenden Schnittstellen (z.B. Position 12 bzw. 38) von pGEM-13zf(+) kloniert und das Konstrukt pGEM-hTRT geschaffen. Die Konstruktion von pGEM-hTRT(Ka) wird dadurch erreicht, daß im Konstrukt pGEM-hTRT eine ca. 20bp Sequenz mit einer ca. 20bp Kassette ausgetauscht wird. Dieser Austausch wird durch rekombinante PCR durchgeführt und ist eine Abwandlung der von Higuchi et al. beschriebenen Verfahren [Higuchi, R. (1988). Nucleic Acid Res 16: 7351-7367; Higuchi, R. (1990). M. Innis A. et al. eds. San Diego, New York, Berkley, Boston, London, Sydney, Tokyo, Toronto, Academic Press, Inc. 177-183]. In einem ersten Schritt werden zwei unabhängige PCR-Reaktionen an pGEM-hTRT durchgeführt: Das Amplifikat aus der 1. PCR-Reaktion ergibt das 5'-Fragment und wird mit geeigneten Restriktionsenzymen zu einem 5'-Fragment verdaut. Das Amplifikat aus der 2. PCR-Reaktion ergibt das 3'-Fragment und wird mit geeigneten Restriktionsenzymen zu einem 3'-Fragment hydrolysiert. Mit T4 Ligase werden die Schnittstellen der 5'- und 3'-Fragmente miteinander zu einem Fragment verbunden, in die korrespondierenden Schnittstellen von pGEM-13zf(+) kloniert und das Konstrukt pGEM-hTRT(Ka) geschaffen. Die Konstruktion von pGEM-hTRT(Kb) und pGEM-hTRT(Kc) wird dadurch erreicht, daß die oben geschaffene ca. 20bp Sequenz im Konstrukt pGEM-hTRT(Ka) jeweils mit einer randomisierten Sequenz von ca. 20bp ausgetauscht wird. In vitro RNA kann dann mit Sp6 RNA Polymerase von pGEM-hTRT(Ka), pGEM-hTRT(Kb) und pGEM-hTRT(Kc) geschaffen werden. Die spezifischen RNAs können dann mit zu o.a. ca. 20bp-Austauschsequenzen bzw. zu der Wildtypsequenz (wt) komplementären Oligonukleotiden O(Ka), O(Kb), O(Kc) und O(wt), nachgewiesen werden. Die weitere Aufbereitung der RNA, wie DNAse Verdau, Reinigung und Kalibrierung erfolgt nach Standardmethoden.

6. Ergebnisse

Die Untersuchungen an Tumorzellinien ergaben, daß die für die katalytische Untereinheit der menschlichen Telomerase kodierende mRNA in unterschiedlichen Mengen in Tumorzellinien bei gleicher Amplifikationsmenge der TCR-, bzw. ß-Actin-Kontrollreaktion nachweisbar war (Abb. 2, 4 und 5). Eine Kontamination mit genomischer DNA konnte durch eine Kontrollreaktion ohne Zugabe von reverser Transkriptase jeweils ausgeschlossen werden.

In vergleichenden Untersuchungen, bei unterschiedlich langen Kultivierungen von Vollblut konnte eindeutig gezeigt werden, daß nach 1 - 5 Tagen Kultur des Vollblutes kein spezifisches RT-PCR Produkt der katalytischen Untereinheit der menschlichen Telomerase mehr detektierbar war. Parallel dazu nahm mit zunehmender Dauer der Kultur die Menge an spezifischer TCR- und Aktin-RNA sehr viel langsamer ab. Das Resultat dieser Kultivierungsuntersuchungen ist, daß offensichtlich kontaminierende Telmomerase-positive Nicht-Tumorzellen schon zwischen Tag 0 und Tag 1 abgestorben sind.

Weiterhin ist unter den beschriebenen RT-PCR-Bedingungen an unterschiedlichen Mengen von Telomerase-positiven Tumorzellen, die katalytische Untereinheit der humanen Telomerase in bis zu 10 Zellen nachweisbar.

Da im Unterschied zum TRAP-Assay in der erfindungsgemäß eingesetzten Reverse-Transkriptase-Polymerase-Kettenreaktion (RT-PCR)-Methode hoch-aufgereinigte RNA zur Amplifikation verwendet wird, kann eine Inhibierung der Taq-Polymerase weitgehend ausgeschlossen werden. Darüber hinaus konnte für die beschriebene RT-PCR-Methode eine Sensitivität für den Nachweis der RNA-Komponente (hTR) sowie der katalytischen Untereinheit (hTRT) der Telomerase von etwa 1 Zelle pro RT-PCR-Ansatz gezeigt werden. Damit bietet die Verwendung der beschriebenen RT-PCR-Methode eine vergleichbare oder höhere Sensitivität des Nachweises von Telomerase-aktiven Tumorzellen. Darüber hinaus wurde gezeigt, daß der Nachweis der hTR- und der hTRT-Expression mit der katalytischen Aktivität der Telomerase korreliert (K. Yashima et al., J. Clin. Pathol. (1997), 50, 110-7).

**Patentansprüche**

1. Verfahren zur Quantifizierung von Tumorzellen in einer Körperflüssigkeit, worin zur Anreicherung der Tumorzellen ein Zellseparationsmedium mit der Körperflüssigkeit überschichtet und zentrifugiert wird, und das Zellseparationsmedium eine Dichte im Bereich von 1,060 bis < 1,065 g/ml aufweist, **dadurch gekennzeichnet, daß**

   (a) die zu untersuchende Probe einem Verfahren zur An- oder Abreicherung von Tumorzellen unterzogen wird und an den an- oder abgereicherten Tumorzellen
   (b) eine Reaktion durchgeführt wird, bei der die für die katalytische Untereinheit der Telomerase kodierende mRNA spezifisch amplifiziert wird, und
   (c) die Menge an amplifizierter Nukleinsäure quantitativ bestimmt wird.

2. Verfahren nach Anspruch 1, **dadurch gekennzeichnet, daß** vor der Amplifizierungsreaktion mit der zu untersuchenden Probe eine reverse Transkriptionsreaktion durchgeführt wird, bei der die in der Probe enthaltene mRNA in cDNA umgeschrieben wird.

3. Verfahren nach Anspruch 1 oder 2, **dadurch gekennzeichnet, daß** mit der zu untersuchenden Probe vor der Umschreibung der mRNA in cDNA eine DNase-Reaktion durchgeführt wird.

4. Verfahren nach einem der Ansprüche 1-3, **dadurch gekennzeichnet, daß** zur quantitativen Bestimmung der Telomerase-kodierenden Nukleinsäure die Amplifikationsprodukte bereits während der Amplifizierung markiert werden, und die Kinetik der Amplifikation kontinuierlich schon während des Amplifikationsvorgangs gemessen wird.

5. Verfahren nach Anspruch 4, **dadurch gekennzeichnet, daß** während der Amplifikation eine für die Amplifikationsprodukte spezifische Sonde vorliegt, die ein für die pro Synthese-Zyklus amplifizierten Produkte proportionales, charakteristisches Signal aussendet.

6. Verfahren nach einem der Ansprüche 1-3, **dadurch gekennzeichnet, daß** zur quantitativen Bestimmung der Telomerase-kodierenden Nukleinsäure mindestens eine, vorzugsweise drei Standard-Nukleinsäuren co-amplifiziert werden, die in unterschiedlichen Konzentrationen zu der zu untersuchenden Probe dazugegeben werden.

7. Verfahren nach einem der Ansprüche 1-6, **dadurch gekennzeichnet, daß** das Amplifizierungsprodukt entweder direkt oder über eine Markierung vorzugsweise über eine radioaktive Markierung, eine Biotin-Markierung, eine Fluoreszenz-Markierung oder eine Elektrochemolumineszenz-Markierung quantifiziert wird.

8. Verfahren nach einem der Ansprüche 1-6, **dadurch gekennzeichnet, daß** das Amplifizierungsprodukt über eine Hybridisierung mit einem markierten Oligonukleotid nachgewiesen wird, wobei die Markierung vorzugsweise eine radioaktive Markierung, eine Biotin-Markierung, eine Fluoreszenz-Markierung oder eine Elektrochemolumineszenz-Markierung ist.

9. Verfahren nach einem der Ansprüche 6-8, **dadurch gekennzeichnet, daß** zur Quantifizierung der zu bestimmenden Telomerase-kodierenden Nukleinsäure die Menge der co-amplifizierten Nukleinsäure bzw. Nukleinsäuren mit der Menge der Telomerase-kodierenden Nukleinsäure verglichen wird.

10. Verfahren nach einem der Ansprüche 1-9, **dadurch gekennzeichnet, daß** es sich bei der zu untersuchenden Probe um peripheres Blut handelt, und daß als positive Kontrolle mit der zu untersuchenden Probe eine Reaktion durchgeführt wird, bei der eine im peripheren Blut vorkommende Nukleinsäure, vorzugsweise die mRNA kodierend für ß-Globin, Glycerinaldehydphosphatdehydrogenase, ß-Aktin oder den T-Zell-Rezeptor, spezifisch amplifiziert und nachgewiesen wird.

11. Verfahren nach Anspruch 1 oder nach einem der Ansprüche 3-10, **dadurch gekennzeichnet, daß** als negative Kontrollen vor der Amplifizierungsreaktion mit der zu untersuchenden Probe keine reverse Transkriptionsreaktion durchgeführt wird und/oder anstelle der Körperflüssigkeit Wasser eingesetzt wird.

12. Verfahren nach einem der Ansprüche 1-11, **dadurch gekennzeichnet, daß** zur Amplifizierung folgende Oligonukleotid-Primer verwendet werden:

5’ CTACCGGAAG AGTGTCTGGA GCAAGTTGCA AAGC 3’ (hTRT1) und/oder
5’ GGCATACCGA CGCACGCAGT ACGTGTTCTG 3’ (hTRT2),

wobei hTRT1 und/oder hTRT2 gegebenenfalls eine Promotorsequenz für eine RNA-Polymerase enthält.

13. Verfahren nach einem der Ansprüche 1-12, **dadurch gekennzeichnet, daß** zur Amplifizierung eine DNA-Polymerase oder eine RNA-Polymerase verwendet wird.

14. Verfahren nach einem der Ansprüche 1-13, **dadurch gekennzeichnet, daß** im Falle der Amplifizierung mit der DNA-Polymerase die Polymerase-Kettenreaktion (PCR) und im Falle der Amplifizierung mit der RNA-Polymerase die isothermale Nukleinsäuresequenz-basierende Amplifikation (NASBA) durchgeführt wird.

15. Verfahren nach einem der Ansprüche 1-14, **dadurch gekennzeichnet, daß** es sich bei der zu untersuchenden Probe um Blut handelt, und daß aus der zu untersuchenden Blutprobe Stammzellen und/oder aktivierte Immunzellen vorzugsweise mittels Immunabsorption abgereichert werden.

16. Verfahren nach einem der Ansprüche 1-15, **dadurch gekennzeichnet, daß** es sich bei der zu untersuchenden Probe um Blut handelt, und daß aus der zu untersuchenden Blutprobe die Tumorzellen vorzugsweise mittels Immunabsorption angereichert werden.

**17.** Verfahren nach Anspruch 1, **dadurch gekennzeichnet, daß** die Zentrifugation bei ca. 1.000 x g über ca. 30 Minuten durchgeführt wird.

**18.** Verfahren nach einem der Ansprüche 1 oder 17, **dadurch gekennzeichnet, daß** das Zellseparationsmedium Percoll oder Ficoll ist.

**19.** verfahren nach einem der Ansprüche 1, 17-18, **dadurch gekennzeichnet, daß** der Körperflüssigkeit vor dem Überschichten eine oder mehrere Substanzen zugegeben werden, die eine Aggregation von Thrombozyten an Tumorzellen verhindern, und/oder die Körperflüssigkeit vor dem Überschichten von Substanzen befreit wird, die eine Aggregation von Thrombozyten an Tumorzellen fördern.

**20.** Verfahren nach einem der Ansprüche 1, 17-19, **dadurch gekennzeichnet, daß** die Körperflüssigkeit peripheres Blut ist.

**21.** Verfahren nach Anspruch 20, **dadurch gekennzeichnet, daß** das periphere Blut in einer gerinnungshemmenden Substanz abgenommen und vor dem Überschichten des Zellseparationsmediums mit einem Verdünnungsmedium bevorzugt im Verhältnis von ca. 1:1 verdünnt wird.

**22.** Verfahren nach Anspruch 20 oder 21, **dadurch gekennzeichnet, daß** das periphere Blut venöses oder arterielles Blut ist.

**23.** Verfahren nach einem der Ansprüche 1, 18-19, **dadurch gekennzeichnet, daß** die Körperflüssigkeit ausgewählt ist aus Lymphe, Urin, Exsudaten, Transudaten, Spinalflüssigkeit, Samenflüssigkeit, Speichel, Flüssigkeiten aus natürlichen oder unnatürlichen Körperhöhlen, Knochenmark und dispergiertem Körpergewebe.

**24.** Verfahren nach einem der Ansprüche 1, 18-23, **dadurch gekennzeichnet, daß** das Zentrifugationsgefäß nach der Zentrifugation und vor der Abnahme der an Tumorzellen angereicherten Interphase stark abgekühlt wird, um ein Vermischen der Zellen in den verschiedenen Schichten zu verhindern.

**25.** Verfahren nach einem der Ansprüche 1, 18-24, **dadurch gekennzeichnet, daß** die Zentrifugation in einem Gefäß durchgeführt wird, das durch eine poröse Barriere, einen Filter oder ein Sieb in ein oberes und ein unteres Kompartiment geteilt ist, wobei das Zellseparationsmedium im unteren Kompartiment vorgelegt und die Körperflüssigkeit in das obere Kompartiment eingebracht wird.

**26.** Verfahren nach Anspruch 25, **dadurch gekennzeichnet, daß** die poröse Barriere, der Filter oder das Sieb eine Dicke von 1-10 mm, vorzugsweise ca. 5 mm aufweisen.

**27.** Verfahren nach Anspruch 25 oder 26, **dadurch gekennzeichnet, daß** die poröse Barriere, der Filter oder das Sieb eine Porengröße von 20-100 $\mu$m, vorzugsweise 20-30 $\mu$m aufweisen.

**28.** Verfahren nach einem der Ansprüche 25-27, **dadurch gekennzeichnet, daß** die poröse Barriere, der Filter oder das Sieb aus einem hydrophoben Material bestehen oder mit einem hydrophoben Material beschichtet sind.

**29.** Verfahren nach einem der Ansprüche 1, 17-28, **dadurch gekennzeichnet, daß** das Zellseparationsmedium einen Farbstoff enthält, der das Zellseparationsmedium von der darüberliegenden Körperflüssigkeit farblich unterscheidbar macht und **dadurch** die Lokalisation der Interphase vereinfacht.

**30.** Verfahren nach einem der Ansprüche 1-29, **dadurch gekennzeichnet, daß** es sich bei der zu untersuchenden Probe um Blut handelt, und daß bei dem genannten Verfahren zum einen eine venöse Blutprobe und zum anderen eine arterielle Blutprobe untersucht wird und die Ergebnisse miteinander verglichen werden.

**31.** Verfahren nach einem der Ansprüche 1-30, **dadurch gekennzeichnet, daß** es sich bei der zu untersuchenden Probe um Blut handelt, und daß bei dem genannten Verfahren zum einen eine Blutprobe aus der Fingerkuppe und zum anderen eine venöse oder arterielle Blutprobe untersucht wird und die Ergebnisse miteinander verglichen werden.

**32.** Verfahren nach einem der Ansprüche 1-31, **dadurch gekennzeichnet, daß** die Tumorzellen von Metastasen, vorzugsweise Mikrometastasen, maligner Tumore stammen.

**33.** Verfahren nach einem der Ansprüche 1-32, **dadurch gekennzeichnet, daß** die Tumorzellen aus einer Gruppe von Zellen metastasierender Tumoren und/oder Neoplasien ausgewählt werden, die von einem T-Zell-Lymphoblastom, T-Zell-Leukämiezellen, chronisch myeloische Leukämiezellen, akute lymphatische Leukämiezellen, chronisch lymphatische Leukämiezellen, Teratokarzinom, Melanom, Lungenkarzinom, Dickdarmkrebs, Brustkrebs, Leberzellkarzinom, Nierentumor, Nebennierentumor, Prostatakarzinom, Neuroblastom, Gehirntumor, Rhabdomyosarkom, Leiomyosarkom und/oder Lymphom abstammen.

**34.** Kit zur Quantifizierung von Tumorzellen in einer Körperflüssigkeit, enthaltend:

(a) ein Oligonukleotid-Primerpaar zur spezifischen Amplifizierung von Telomerase-kodierender mRNA, und
(b) zusätzlich ein Zellseparationsmedium, das eine Dichte im Bereich von 1,060 bis < 1,065 g/ml aufweist, sowie gegebenenfalls ein Zentrifugationsgefäß.

**35.** Kit nach Anspruch 34, **dadurch gekennzeichnet, daß** das Oligonukleotid-Primerpaar gemäß (a) folgende Sequenzen hat:

5' CTACCGGAAG AGTGTCTGGA GCAAGTTGCA AAGC 3' (hTRT1) und/oder
5' GGCATACCGA CGCACGCAGT ACGTGTTCTG 3' (hTRT2),

wobei hTRT1 und/oder hTRT2 gegebenenfalls eine Promotorsequenz für eine RNA-Polymerase enthält.

**36.** Kit nach einem der Ansprüche 34 oder 35, **dadurch gekennzeichnet, daß** er zusätzlich (b) eine Standard-Nukleinsäure bzw. Standard-Nukleinsäuren zur Co-Amplifikation enthält.

**37.** Kit nach einem der Ansprüche 34-36, **dadurch gekennzeichnet, daß** er zusätzlich ein markiertes Oligonukleotid zum Nachweis der amplifizierten Nukleinsäure der zu bestimmenden Probe und/oder ein oder mehrere markierte Oligonukleotide zum Nachweis der co-amplifizierten Standard-Nukleinsäure bzw. Standard-Nukleinsäuren enthält, insbesondere ein Oligonukleotid mit der Sequenz:

5' CGTTCTGGCT CCCACGACGT AGTC 3' (hTRT o)

und/oder die entsprechende reverse komplementäre Sequenz davon.

**38.** Kit nach einem der Ansprüche 34-37, **dadurch gekennzeichnet, daß** er zusätzlich eine reverse Transkriptase, eine DNA-Polymerase, vorzugsweise eine Taq-Polymerase, eine DNase und/oder geeignete Puffer und gegebenenfalls markierte Nukleotide und gegebenenfalls zur Abreicherung von Stammzellen und/oder aktivierten Immunzellen und/oder zur Anreicherung von Tumorzellen geeignete Mittel enthält.

**39.** Kit nach einem der Ansprüche 34-38, **dadurch gekennzeichnet, daß** er zusätzlich eine reverse Transkriptase, eine RNA-Polymerase, vorzugsweise eine T7 RNA-Polymerase, eine RNase H, eine DNase und/oder geeignete Puffer und gegebenenfalls markierte Nukleotide und gegebenenfalls zur Abreicherung von Stammzellen und/oder aktivierten Immunzellen und/oder zur Anreicherung von Tumorzellen geeignete Mittel enthält.

**40.** Kit nach Anspruch 34, worin das Zentrifugationsgefäß eine poröse Barriere, einen Filter oder ein Sieb mit einer Dicke von 1-10 mm, vorzugsweise ca. 5 mm aufweist, die das Zentrifugationsgefäß in ein oberes und ein unteres Kompartiment unterteilen.

**41.** Kit nach Anspruch 40, worin die poröse Barriere, der Filter oder das Sieb eine Porengröße von 20-100 $\mu$m, vorzugsweise 20-30 $\mu$m aufweisen.

**42.** Kit nach Anspruch 40 oder 41, worin sich das Zellseparationsmedium im unteren Kompartiment des Zentrifugationsgefäßes befindet.

## Claims

1. Method for the quantification of tumor cells in a body fluid, where, for enriching the tumor cells, a cell separation medium is covered with a layer of the body fluid and centrifuged and the cell separation medium has a density in the range from 1.060 to < 1.065 g/ml, **characterized in that**

    (a) the sample to be investigated is subjected to an enrichment or depletion of tumor cells and **in that** on the enriched or depleted tumor cells
    (b) a reaction is carried out, in which the mRNA coding for the catalytic subunit of telomerase is specifically amplified; and
    (c) the amount of amplified nucleic acid is determined quantitatively.

2. Method according to Claim 1, **characterized in that** a reverse transcription reaction in which the mRNA contained in the sample is transcribed into cDNA is carried out before the amplification reaction with the sample to be investigated.

3. Method according to Claim 1 or 2, **characterized in that** a DNase reaction is carried out with the sample to be investigated before the transcription of the mRNA into cDNA.

4. Method according to any one of Claims 1 - 3, **characterized in that**, for quantitative determination of the telomerase-coding nucleic acid, the amplification products are labeled even during amplification and the amplification kinetics are measured continuously even during the amplification process.

5. Method according to Claim 4, **characterized in that** a probe which is specific for the amplification products, and which emits a characteristic signal proportional to the products amplified per synthesis cycle, is present during amplification.

6. Method according to any one of Claims 1 - 3, **characterized in that**, for quantitative determination of the telomerase-encoding nucleic acid, at least one, preferably three standard nucleic acids are coamplified and are added in different concentrations to the sample to be investigated.

7. Method according to any one of Claims 1 - 6, **characterized in that** the amplification product is quantified either directly or via a label, wherein the label is preferably a radioactive label, a biotin label, a fluorescent label or an electrochemoluminescent label.

8. Method according to any one of Claims 1 - 6, **characterized in that** the amplification product is detected via hybridization with a labeled oligonucleotide, wherein the label is preferably a radioactive label, a biotin label, a fluorescent label or an electrochemoluminescent label.

9. Method according to any one of Claims 6 - 8, **characterized in that**, to quantify the telomerase-encoding nucleic acid to be determined, the amount of coamplified nucleic acid or nucleic acids is compared with the amount of telomerase-encoding nucleic acid.

10. Method according to any one of Claims 1 - 9, **characterized in that** the sample to be investigated is peripheral blood, and **in that** a reaction is carried out with the sample to be investigated as positive control, in which a nucleic acid which occurs in peripheral blood, preferably the mRNA coding for β-globin, glyceraldehyde-phosphate dehydrogenase, β-actin or T-cell receptor, is specifically amplified and detected.

11. Method according to Claim 1 and or any of Claims 3 - 10, **characterized in that**, as negative controls, no reverse transcription reaction is carried out before the amplification reaction with the sample to be investigated and/or water is employed in place of the body fluid.

12. Method according to any one of Claims 1 - 11, **characterized in that** the following oligonucleotide primers are used for the amplification:

    5' CTACCGGAAG AGTGTCTGGA GCAAGTTGCA AAGC 3' (hTRT1) and/or
    5' GGCATACCGA CGCACGCAGT ACGTGTTCTG 3' (hTRT2),

wherein hTRT1 and/or hTRT2 optionally comprise a promoter sequence for an RNA polymerase.

**13.** Method according to any one of Claims 1 - 12, **characterized in that** a DNA polymerase or an RNA polymerase is used for the amplification.

**14.** Method according to any one of Claims 1 - 13, **characterized in that** in case the DNA polymerase is used for the amplification the polymerase chain reaction (PCR) is carried out and in case the RNA polymerase is used for the amplification the isothermal nucleic acid sequence-based amplification (NASBA) is carried out.

**15.** Method according to any one of Claims 1 - 14, **characterized in that** the sample to be investigated is blood, and **in that** the blood sample to be investigated is depleted in stem cells and/or activated immune cells, preferably by immunoabsorption.

**16.** Method according to any one of Claims 1 - 15, **characterized in that** the sample to be investigated is blood, and **in that** the tumor cells from the blood sample to be investigated are concentrated, preferably by immunoabsorption.

**17.** Method according to Claim 1, **characterized in that** the centrifugation is carried out at about 1000 x g for about 30 minutes.

**18.** Method according to Claim 1 or 17, **characterized in that** the cell separation medium used is Percoll™ or Ficoll™.

**19.** Method according to any one of Claims 1, 17 - 18, **characterized in that** the body fluid is, prior to being applied as a covering layer, admixed with one or more substances which prevent aggregation of thrombocytes to tumor cells and/or the body fluid is, prior to being applied as a covering layer, freed of substances which promote aggregation of thrombocytes to tumor cells.

**20.** Method according to any one of Claims 1, 17 - 19, **characterized in that** the body fluid is peripheral blood.

**21.** Method according to Claim 20, **characterized in that** the peripheral blood is drawn in an anticoagulant substance and, prior to covering the cell separation medium, diluted with a diluent, preferably in a ratio of about 1:1.

**22.** Method according to Claim 20 or 21, **characterized in that** the peripheral blood is venous or arterial blood.

**23.** Method according to any one of Claims 1, 18 - 19, **characterized in that** the body fluid is selected from lymph, urine, exudates, transudates, spinal fluid, seminal fluid, saliva, fluids from natural or unnatural body cavities, bone marrow and dispersed body tissue.

**24.** Method according to any one of Claims 1, 18 - 23, **characterized in that** the centrifugation vessel is, after centrifugation and before the tumor-cell-enriched interphase is removed, cooled intensively to prevent mixing of the cells in the different layers.

**25.** Method according to any one of Claims 1, 18 - 24, **characterized in that** the centrifugation is carried out in a vessel which is divided by a porous barrier, a filter or a sieve into an upper and a lower compartment, where the cell separation medium is initially charged in the lower compartment and the body fluid is introduced into the upper compartment.

**26.** Method according to Claim 25, **characterized in that** the porous barrier, the filter or the sieve has a thickness of 1-10 mm, preferably about 5 mm.

**27.** Method according to Claim 25 or 26, **characterized in that** the porous barrier, the filter or the sieve has a pore size of 20-100 μm, preferably 20-30 μm.

**28.** Method according to any one of Claims 25 - 27, **characterized in that** the porous barrier, the filter or the sieve is made of a hydrophobic material or coated with a hydrophobic material.

**29.** Method according to any one of Claims 1, 17 - 28, **characterized in that** the cell separation medium contains a dye which makes the color of the cell separation medium distinguishable from that of the supernatant body fluid, thus simplifying the localization of the interphase.

**30.** Method according to any one of Claims 1 - 29, **characterized in that** the sample to the investigated is blood and **in that** a venous blood sample and an arterial blood sample are investigated and the results obtained are compared with one another.

**31.** Method according to any one of Claims 1 - 30, **characterized in that** the sample to be investigated is blood and **in that** a blood sample from the finger pulp and a venous or arterial blood sample are investigated, and the results obtained are compared with one another.

**32.** Method according to any one of Claims 1 - 31, **characterized in that** the tumor cells are derived from metastases, preferably micrometastases, of malignant tumors.

**33.** Method according to any of Claims 1 - 32, **characterized in that** the tumor cells are selected from a group of cells of metastasizing tumors and/or neoplasms which are derived from a T-cell lymphoblastoma, T-cell leukemia cells, chronic myeloid leukemia cells, acute lymphatic leukemia cells, chronic lymphatic leukemia cells, teratocarcinoma, melanoma, carcinoma of the lung, large intestine cancer, breast cancer, hepatocellular carcinoma, kidney tumor, adrenal tumor, prostate carcinoma, neuroblastoma, brain tumor, rhabdomyosarcoma, leiomyosarcoma and/or lymphoma.

**34.** Kit for the quantification of tumor cells in a body fluid, comprising:

(a) an oligonucleotide primer pair for specific amplification of telomerase-encoding mRNA, and
(b) additionally comprises a cell separation medium having a density in the range of from 1.060 to < 1.065 g/ml as well as optionally a centrifugation vessel.

**35.** Kit according to Claim 34, **characterized in that** the oligonucleotide primer pair specified in (a) has the following sequences:

5' CTACCGGAAG AGTGTCTGGA GCAAGTTGCA AAGC 3' (hTRT1) and/or
5' GGCATACCGA CGCACGCAGT ACGTGTTCTG 3' (hTRT2),

wherein hTRT1 and/or hTRT2 further comprises a promoter sequence for an RNA polymerase.

**36.** Kit according to Claim 34 or 35, **characterized in that** it additionally comprises (b) a standard nucleic acid or standard nucleic acids for coamplification.

**37.** Kit according to any one of Claims 34 - 36, **characterized in that** it additionally comprises a labeled oligonucleotide for detecting the amplified nucleic acid of the sample to be determined and/or one or more labeled oligonucleotides for detecting the coamplified standard nucleic acid or standard nucleic acids, in particular an oligonucleotide having the sequence:

5' CGTTCTGGCT CCCACGACGT AGTC 3' (hTRT o)

and/or the corresponding reverse complementary sequence thereof.

**38.** Kit according to any one of Claims 34 - 37, **characterized in that** it additionally comprises a reverse transcriptase, a DNA polymerase, preferably a Taq polymerase, a DNase and/or suitable buffers and, optionally, labeled nucleotides and, optionally, means suitable for the depletion of stem cells and/or activated immune cells and/or means suitable for the concentration of tumor cells.

**39.** Kit according to any of Claims 34 - 38, **characterized in that** it additionally comprises a reverse transcriptase, a RNA polymerase, preferably a T7 RNA polymerase, an RNase H, a DNase and/or suitable buffers and, optionally, labeled nucleotides and, optionally, means suitable for the depletion of stem cells and/or activated immune cells and/or means suitable for the concentration of tumor cells.

**40.** Kit according to of Claim 34, **characterized in that** the centrifugation vessel has a porous barrier, a filter or a sieve of a thickness of 1-10 mm, preferably about 5 mm, which divides the centrifugation vessel into an upper and a lower compartment.

**41.** Kit according to Claim 40, **characterized in that** the porous barrier, the filter or the sieve has a pore size of 20-100 μm, preferably 20-30 μm.

**42.** Kit according to Claim 40 or 41, **characterized in that** the cell separation medium is in the lower compartment of the centrifugation vessel.

## Revendications

**1.** Procédé de quantification de cellules tumorales dans un fluide corporel, dans lequel pour l'enrichissement des cellules tumorales, un milieu de séparation cellulaire est recouvert par le fluide corporel et centrifugé et le milieu de séparation cellulaire présente une densité de l'ordre de 1,060 à < 1,065 g/ml, **caractérisé en ce que**

(a) l'échantillon à analyser est soumis à un procédé d'enrichissement ou d'appauvrissement de cellules tumorales et sur les cellules tumorales enrichies ou appauvries,
(b) une réaction est réalisée, dans laquelle l'ARNm codant pour la sous-unité catalytique de la télomérase est amplifié sélectivement, et
(c) la quantité d'acide nucléique amplifiée est déterminée quantitativement.

**2.** Procédé selon la revendication 1, **caractérisé en ce que**, avant la réaction d'amplification avec l'échantillon à analyser, une réaction de transcription inverse est réalisée, dans laquelle l'ARNm contenu dans l'échantillon est transcrit en ADNc.

**3.** Procédé selon la revendication 1 ou 2, **caractérisé en ce que**, avec l'échantillon à analyser, avant la transcription de l'ARNm en ADNc, une réaction de DNase est réalisée.

**4.** Procédé selon l'une des revendications 1 à 3, **caractérisé en ce que**, pour la détermination quantitative de l'acide nucléique codant pour la télomérase, les produits d'amplification sont déjà marqués pendant l'amplification et la cinétique d'amplification est déjà mesurée en continu pendant le processus d'amplification.

**5.** Procédé selon la revendication 4, **caractérisé en ce que**, pendant l'amplification, on dispose d'une sonde spécifique des produits d'amplification qui émet un signal caractéristique, proportionnel des produits amplifiés par cycle de synthèse.

**6.** Procédé selon l'une des revendications 1 à 3, **caractérisé en ce que**, pour la détermination quantitative de l'acide nucléique codant pour la télomérase, au moins un, de préférence trois acide(s) nucléique(s) standard sont co-amplifiés, qui sont ajoutés en différentes concentrations, à l'échantillon à analyser.

**7.** Procédé selon l'une des revendications 1 à 6, **caractérisé en ce que** le produit d'amplification est quantifié soit directement, soit par un marquage, de préférence par un marquage radioactif, un marquage à la biotine, un marquage par fluorescence ou un marquage par électrochimioluminescence.

**8.** Procédé selon l'une des revendications 1 à 6, **caractérisé en ce que** le produit d'amplification est détecté par une hybridation avec un oligonucléotide marqué, le marquage étant de préférence un marquage radioactif, un marquage à la biotine, un marquage par fluorescence ou un marquage par électrochimioluminescence.

**9.** Procédé selon l'une des revendications 6 à 8, **caractérisé en ce que**, pour la quantification de l'acide nucléique codant pour la télomérase à déterminer, la quantité d'acide nucléique ou d'acides nucléiques co-amplifié(s) est comparée à la quantité d'acide nucléique codant pour la télomérase.

**10.** Procédé selon l'une des revendications 1 à 9, **caractérisé en ce que** l'échantillon à analyser est du sang périphérique et **en ce que**, comme témoin positif, une réaction est réalisée avec l'échantillon à analyser, dans lequel un acide nucléique présent dans le sang périphérique, de préférence, l'ARNm codant pour la bêta-globine, la glycérinaldéhyde-phosphate-déshydrogénase, la bêta-actine ou le récepteur des lymphocytes T est amplifié et détecté spécifiquement.

**11.** Procédé selon la revendication 1 ou selon l'une des revendications 3 à 10, **caractérisé en ce que**, comme témoin négatif avant la réaction d'amplification avec l'échantillon à analyser, aucune réaction de transcription inverse n'est réalisée et/ou on utilise à la place l'eau du fluide corporel.

12. Procédé selon l'une des revendications 1 à 11, **caractérisé en ce que**, pour l'amplification, on utilise les amorces d'oligonucléotides suivantes :

5' CTACCGGAAG AGTGTCTGGA GCAAGTTGCA AAGC 3' (hTRT1) et/ou
5' GGCATACCGA CGCACGCAGT ACGTGTTCTG 3' (hTRT2),

où hTRT1 et/ou hTRT2 contiennent éventuellement une séquence promoteur d'une ARN-polymérase.

13. Procédé selon l'une des revendications 1 à 12, **caractérisé en ce que** pour l'amplification, on utilise une ADN-polymérase ou une ARN-polymérase.

14. Procédé selon l'une des revendications 1 à 13, **caractérisé en ce que**, dans le cas de l'amplification avec l'ADN-polymérase, la réaction en chaîne polymérase (PCR) et dans le cas de l'amplification avec l'ARN polymérase, l'amplification se basant sur la séquence d'acide nucléique isothermique (NASBA) est réalisée.

15. Procédé selon l'une des revendications 1 à 14, **caractérisé en ce que** l'échantillon à analyser est du sang et **en ce que**, à partir de l'échantillon de sang à analyser, des cellules souches et/ou des cellules immunitaires activées sont appauvries de préférence par immuno-absorption.

16. Procédé selon l'une des revendications 1 à 15, **caractérisé en ce que** l'échantillon à analyser est du sang, et **en ce que**, à partir de l'échantillon de sang à analyser, les cellules tumorales sont enrichies de préférence par immuno-absorption.

17. Procédé selon la revendication 1, **caractérisé en ce que** la centrifugation est réalisée à environ 1,000 x g pendant environ 30 minutes.

18. Procédé selon l'une des revendications 1 ou 17, **caractérisé en ce que** le milieu de séparation cellulaire est le Percoll ou le Ficoll.

19. Procédé selon l'une des revendications 1, 17 à 18, **caractérisé en ce que** l'on ajoute au fluide corporel, avant la superposition, une ou plusieurs substances qui empêchent une agrégation plaquettaire sur les cellules tumorales et/ou le fluide corporel est libéré avant la superposition de substances qui stimulent une agrégation plaquettaire sur les cellules tumorales.

20. Procédé selon l'une des revendications 1, 17 à 19, **caractérisé en ce que** le fluide corporel est du sang périphérique.

21. Procédé selon la revendication 20, **caractérisé en ce que** le sang périphérique est appauvri dans une substance inhibant la coagulation et avant la superposition du milieu de séparation cellulaire, avec un milieu de dilution, de préférence en un rapport d'environ 1:1.

22. Procédé selon la revendication 20 ou 21, **caractérisé en ce que** le sang périphérique est du sang veineux ou du sang artériel.

23. Procédé selon l'une des revendications 1, 18 à 19, **caractérisé en ce que** le fluide corporel est choisi parmi la lymphe, l'urine, les exsudats, les transsudats, le liquide céphalorachidien, le sperme, la salive, les fluides de cavités corporelles naturelles ou non naturelles, la moelle osseuse et les tissus corporels dispersés.

24. Procédé selon l'une des revendications 1, 18 à 23, **caractérisé en ce que** le récipient de centrifugation après la centrifugation et avant l'appauvrissement de l'interphase enrichie en cellules tumorales est fortement refroidi pour empêcher un mélange des cellules dans les différentes couches.

25. Procédé selon l'une des revendications 1, 18 à 24, **caractérisé en ce que** la centrifugation est réalisée dans un récipient qui est séparé par une barrière poreuse, un filtre ou un tamis en un compartiment supérieur et un compartiment inférieur, le milieu de séparation cellulaire étant placé dans le compartiment inférieur et le fluide corporel étant inséré dans le compartiment supérieur.

**26.** Procédé selon la revendication 25, **caractérisé en ce que** la barrière poreuse, le filtre ou le tamis présentent une épaisseur de 1 à 10 mm, de préférence d'environ 5 mm.

**27.** Procédé selon la revendication 25 ou 26, **caractérisé en ce que** la barrière poreuse, le filtre ou le tamis présentent une taille de pore de 20 à 100 μm, de préférence de 20 à 30 μm.

**28.** Procédé selon l'une des revendications 25 à 27, **caractérisé en ce que** la barrière poreuse, le filtre ou le tamis est constitué(e) d'un matériau hydrophobe ou est revêtu(e) d'un matériau hydrophobe.

**29.** Procédé selon l'une des revendications 1, 17 à 28, **caractérisé en ce que** le milieu de séparation cellulaire contient un colorant qui permet de distinguer par coloration le milieu de séparation cellulaire du fluide corporel sous-jacent et ainsi, simplifie la localisation de l'interphase.

**30.** Procédé selon l'une des revendications 1 à 29, **caractérisé en ce que** l'échantillon à analyser est du sang et **en ce que**, dans le procédé cité, d'une part, un échantillon de sang veineux et d'autre part, un échantillon de sang artériel sont analysés et les résultats sont comparés les uns aux autres.

**31.** Procédé selon l'une des revendications 1 à 30, **caractérisé en ce que** l'échantillon à analyser est du sang et **en ce que**, dans le procédé cité, d'une part un échantillon de sang de la pointe des doigts et d'autre part, un échantillon de sang veineux ou artériel sont analysés et les résultats sont comparés les uns aux autres.

**32.** Procédé selon l'une des revendications 1 à 31, **caractérisé en ce que** les cellules tumorales proviennent de métastases, de préférence de micrométastases, de tumeurs malignes.

**33.** Procédé selon l'une des revendications 1 à 32, **caractérisé en ce que** les cellules tumorales sont choisies dans un groupe de cellules de tumeurs métastasiques et/ou de néoplasies, qui proviennent d'un lymphoblastome à lymphocytes T, de cellules leucémiques à lymphocytes T, de cellules leucémiques myéloïdes chroniques, de cellules leucémiques lymphatiques aiguës, de cellules leucémiques lymphatiques chroniques, de tétratocarcinome, de mélanome, le carcinome pulmonaire, de cancer du côlon, de cancer du sein, de carcinome hépatique, de tumeur rénale, de tumeur des surrénales, de carcinome prostatique, de neuroblastome, de tumeur cérébrale, de rhabdomyosarcome, le léiomyosarcome et/ou de lymphomes.

**34.** Trousse pour la quantification de cellules tumorales dans un fluide corporel, contenant :

(a) une paire d'amorces d'oligonucléotide pour l'amplification spécifique d'ARNm codant pour la télomérase, et
(b) en outre, un milieu de séparation cellulaire qui présente une épaisseur de l'ordre de 1,060 à < 1,065 g/ml et éventuellement un récipient de centrifugation.

**35.** Trousse selon la revendication 34, **caractérisée en ce que** la paire d'amorce d'oligonucléotide selon le point (a) a les séquences suivantes :

5' CTACCGGAAG AGTGTCTGGA GCAAGTTGCA AAGC 3' (hTRT1) et/ou
5' GGCATACCGA CGCACGCAGT ACGTGTTCTG 3' (hTRT2),

où hTRT1 et/ou hTRT2 contiennent éventuellement une séquence promoteur d'une ARN-polymérase.

**36.** Trousse selon l'une des revendications 34 ou 35, **caractérisée en ce qu'**elle contient en outre, un acide nucléique standard ou des acides nucléiques standard pour la coamplification.

**37.** Trousse selon l'une des revendications 34 à 36, **caractérisée en ce qu'**elle contient en outre un oligonucléotide marqué pour la détection de l'acide nucléique amplifié de l'échantillon à déterminer et/ou un ou plusieurs oligonucléotides marqués pour la détection de l'acide nucléique ou des acides nucléiques standard co-amplifiés, en particulier un oligonucléotide présentant la séquence:

5' CGTTCTGGCT CCCACGACGT AGTC 3' (hTRT 0)

et/ou la séquence complémentaire inverse correspondante de celle-ci.

**38.** Trousse selon l'une des revendications 34 à 37, **caractérisée en ce qu'**elle contient en outre une transcriptase inverse, une ADN-polymérase, de préférence une Taq-polymérase, une DNase et/ou un tampon approprié et éventuellement des nucléotides marqués et éventuellement un milieu approprié pour l'appauvrissement de cellules souches et/ou de cellules immunitaires activés et/ou pour l'enrichissement de cellules tumorales.

**39.** Trousse selon l'une des revendications 34 à 38, **caractérisée en ce qu'**elle contient en outre une transcriptase inverse, une ARN-polymérase, de préférence une ARN T7-polymérase, une RNase H, une DNase et/ou un tampon approprié et éventuellement des nucléotides marqués et éventuellement un milieu approprié pour l'appauvrissement de cellules souches et/ou de cellules immunitaires activées et/ou pour l'enrichissement de cellules tumorales.

**40.** Trousse selon la revendication 34, dans laquelle le récipient de centrifugation présente une barrière poreuse, un filtre ou un tamis présentant une épaisseur de 1 à 10 mm, de préférence d'environ 5 mm, qui sépare le récipient de centrifugation en un compartiment supérieur et un compartiment inférieur.

**41.** Trousse selon la revendication 40, dans laquelle la membrane poreuse, le filtre ou le tamis présentent une taille de pores de 20 à 100 $\mu$m, de préférence de 20 à 30 $\mu$m.

**42.** Trousse selon la revendication 40 ou 41, dans laquelle le milieu de séparation cellulaire se trouve dans le compartiment inférieur du récipient de centrifugation.

1 GCAGCGCTGC GTCCTGCTGC GCACGTGGGA AGCCCTGGCC CCGGCCACCC CCGCGATGCC
61 GCGCGCTCCC CGCTGCCGAG CCGTGCGCTC CCTGCTGCGC AGCCACTACC GCGAGGTGCT
121 GCCGCTGGCC ACGTTCGTGC GGCGCCTGGG GCCCCAGGGC TGGCGGCTGG TGCAGCGCGG
181 GGACCCGGCG GCTTTCCGCG CGCTGGTGGC CCAGTGCCTG GTGTGCGTGC CCTGGGACGC
241 ACGGCCGCCC CCCGCCGCCC CCTCCTTCCG CCAGGTGTCC TGCCTGAAGG AGCTGGTGGC
301 CCGAGTGCTG CAGAGGCTGT GCGAGCGCGG CGCGAAGAAC GTGCTGGCCT TCGGCTTCGC
361 GCTGCTGGAC GGGGCCCGCG GGGGCCCCCC CGAGGCCTTC ACCACCAGCG TGCGCAGCTA
421 CCTGCCCAAC ACGGTGACCG ACGCACTGCG GGGGAGCGGG GCGTGGGGGC TGCTGCTGCG
481 CCGCGTGGGC GACGACGTGC TGGTTCACCT GCTGGCACGC TGCGCGCTCT TTGTGCTGGT
541 GGCTCCCAGC TGCGCCTACC AGGTGTGCGG GCCGCCGCTG TACCAGCTCG GCGCTGCCAC
601 TCAGGCCCGG CCCCCGCCAC ACGCTAGTGG ACCCCGAAGG CGTCTGGGAT GCGAACGGGC
661 CTGGAACCAT AGCGTCAGGG AGGCCGGGGT CCCCCTGGGC CTGCCAGCCC CGGGTGCGAG
721 GAGGCGCGGG GGCAGTGCCA GCCGAAGTCT GCCGTTGCCC AAGAGGCCCA GGCGTGGCGC
781 TGCCCCTGAG CCGGAGCGGA CGCCCGTTGG GCAGGGGTCC TGGGCCCACC CGGGCAGGAC
841 GCGTGGACCG AGTGACCGTG GTTTCTGTGT GGTGTCACCT GCCAGACCCG CCGAAGAAGC
901 CACCTCTTTG GAGGGTGCGC TCTCTGGCAC GCGCCACTCC CACCCATCCG TGGGCCGCCA
961 GCACCACGCG GGCCCCCCAT CCACATCGCG GCCACCACGT CCCTGGGACA CGCCTTGTCC
1021 CCCGGTGTAC GCCGAGACCA AGCACTTCCT CTACTCCTCA GGCGACAAGG AGCAGCTGCG
1081 GCCCTCCTTC CTACTCAGCT CTCTGAGGCC CAGCCTGACT GGCGCTCGGA GGCTCGTGGA
1141 GACCATCTTT CTGGGTTCCA GGCCCTGGAT GCCAGGGACT CCCCGCAGGT TGCCCCGCCT
1201 GCCCCAGCGC TACTGGCAAA TGCGGCCCCT GTTTCTGGAG CTGCTTGGGA ACCACGCGCA
1261 GTGCCCCTAC GGGGTGCTCC TCAAGACGCA CTGCCCGCTG CGAGCTGCGG TCACCCCAGC
1321 AGCCGGTGTC TGTGCCCGGG AGAAGCCCCA GGGCTCTGTG GCGGCCCCCG AGGAGGAGGA
1381 CACAGACCCC CGTCGCCTGG TGCAGCTGCT CCGCCAGCAC AGCAGCCCCT GGCAGGTGTA
1441 CGGCTTCGTG CGGGCCTGCC TGCGCCGGCT GGTGCCCCCA GGCCTCTGGG GCTCCAGGCA
1501 CAACGAACGC CGCTTCCTCA GGAACACCAA GAAGTTCATC TCCCTGGGGA AGCATGCCAA
1561 GCTCTCGCTG CAGGAGCTGA CGTGGAAGAT GAGCGTGCGG GACTGCGCTT GGCTGCGCAG
1621 GAGCCCAGGG GTTGGCTGTG TTCCGGCCGC AGAGCACCGT CTGCGTGAGG AGATCCTGGC
1681 CAAGTTCCTG CACTGGCTGA TGAGTGTGTA CGTCGTCGAG CTGCTCAGGT CTTTCTTTTA
1741 TGTCACGGAG ACCACGTTTC AAAAGAACAG GCTCTTTTTC TACCGGAAGA GTGTCTGGAG
1801 CAAGTTGCAA AGCATTGGAA TCAGACAGCA CTTGAAGAGG GTGCAGCTGC GGGAGCTGTC
1861 GGAAGCAGAG GTCAGGCAGC ATCGGGAAGC CAGGCCCGCC CTGCTGACGT CCAGACTCCG
1921 CTTCATCCCC AAGCCTGACG GGCTGCGGCC GATTGTGAAC ATGGACTACG TCGTGGGAGC
1981 CAGAACGTTC CGCAGAGAAA AGAGGGCCGA GCGTCTCACC TCGAGGGTGA AGGCACTGTT
2041 CAGCGTGCTC AACTACGAGC GGGCGCGGCG CCCCGGCCTC CTGGGCGCCT CTGTGCTGGG
2101 CCTGGACGAT ATCCACAGGG CCTGGCGCAC CTTCGTGCTG CGTGTGCGGG CCCAGGACCC
2161 GCCGCCTGAG CTGTACTTTG TCAAGGTGGA TGTGACGGGC GCGTACGACA CCATCCCCCA
2221 GGACAGGCTC ACGGAGGTCA TCGCCAGCAT CATCAAACCC CAGAACACGT ACTGCGTGCG
2281 TCGGTATGCC GTGGTCCAGA AGGCCGCCCA TGGGCACGTC CGCAAGGCCT TCAAGAGCCA
2341 CGTCTCTACC TTGACAGACC TCCAGCCGTA CATGCGACAG TTCGTGGCTC ACCTGCAGGA
2401 GACCAGCCCG CTGAGGGATG CCGTCGTCAT CGAGCAGAGC TCCTCCCTGA ATGAGGCCAG
2461 CAGTGGCCTC TTCGACGTCT TCCTACGCTT CATGTGCCAC CACGCCGTGC GCATCAGGGG
2521 CAAGTCCTAC GTCCAGTGCC AGGGGATCCC GCAGGGCTCC ATCCTCTCCA CGCTGCTCTG
2581 CAGCCTGTGC TACGGCGACA TGGAGAACAA GCTGTTTGCG GGGATTCGGC GGGACGGGCT
2641 GCTCCTGCGT TTGGTGGATG ATTTCTTGTT GGTGACACCT CACCTCACCC ACGCGAAAAC
2701 CTTCCTCAGG ACCCTGGTCC GAGGTGTCCC TGAGTATGGC TGCGTGGTGA ACTTGCGGAA
2761 GACAGTGGTG AACTTCCCTG TAGAAGACGA GGCCCTGGGT GGCACGGCTT TTGTTCAGAT
2821 GCCGGCCCAC GGCCTATTCC CCTGGTGCGG CCTGCTGCTG GATACCCGGA CCCTGGAGGT
2881 GCAGAGCGAC TACTCCAGCT ATGCCCGGAC CTCCATCAGA GCCAGTCTCA CCTTCAACCG
2941 CGGCTTCAAG GCTGGGAGGA ACATGCGTCG CAAACTCTTT GGGGTCTTGC GGCTGAAGTG
3001 TCACAGCCTG TTTCTGGATT TGCAGGTGAA CAGCCTCCAG ACGGTGTGCA CCAACATCTA
3061 CAAGATCCTC CTGCTGCAGG CGTACAGGTT TCACGCATGT GTGCTGCAGC TCCCATTTCA
3121 TCAGCAAGTT TGGAAGAACC CCACATTTTT CCTGCGCGTC ATCTCTGACA CGGCCTCCCT
3181 CTGCTACTCC ATCCTGAAAG CCAAGAACGC AGGGATGTCG CTGGGGGCCA AGGGCGCCGC
3241 CGGCCCTCTG CCCTCCGAGG CCGTGCAGTG GCTGTGCCAC CAAGCATTCC TGCTCAAGCT
3301 GACTCGACAC CGTGTCACCT ACGTGCCACT CCTGGGGTCA CTCAGGACAG CCCAGACGCA

Abb. 1a

```
3361 GCTGAGTCGG AAGCTCCCGG GGACGACGCT GACTGCCCTG GAGGCCGCAG CCAACCCGGC
3421 ACTGCCCTCA GACTTCAAGA CCATCCTGGA CTGATGGCCA CCCGCCCACA GCCAGGCCGA
3481 GAGCAGACAC CAGCAGCCCT GTCACGCCGG GCTCTACGTC CCAGGGAGGG AGGGGCGGCC
3541 CACACCCAGG CCCGCACCGC TGGGAGTCTG AGGCCTGAGT GAGTGTTTGG CCGAGGCCTG
3601 CATGTCCGGC TGAAGGCTGA GTGTCCGGCT GAGGCCTGAG CGAGTGTCCA GCCAAGGGCT
3661 GAGTGTCCAG CACACCTGCC GTCTTCACTT CCCCACAGGC TGGCGCTCGG CTCCACCCCA
3721 GGGCCAGCTT TTCCTCACCA GGAGCCCGGC TTCCACTCCC CACATAGGAA TAGTCCATCC
3781 CCAGATTCGC CATTGTTCAC CCCTCGCCCT GCCCTCCTTT GCCTTCCACC CCCACCATCC
3841 AGGTGGAGAC CCTGAGAAGG ACCCTGGGAG CTCTGGGAAT TTGGAGTGAC CAAAGGTGTG
3901 CCCTGTACAC AGGCGAGGAC CCTGCACCTG GATGGGGGTC CCTGTGGGTC AAATTGGGGG
3961 GAGGTGCTGT GGGAGTAAAA TACTGAATAT ATGAGTTTTT CAGTTTTGAA AAAAA
```

**Abb. 1b**

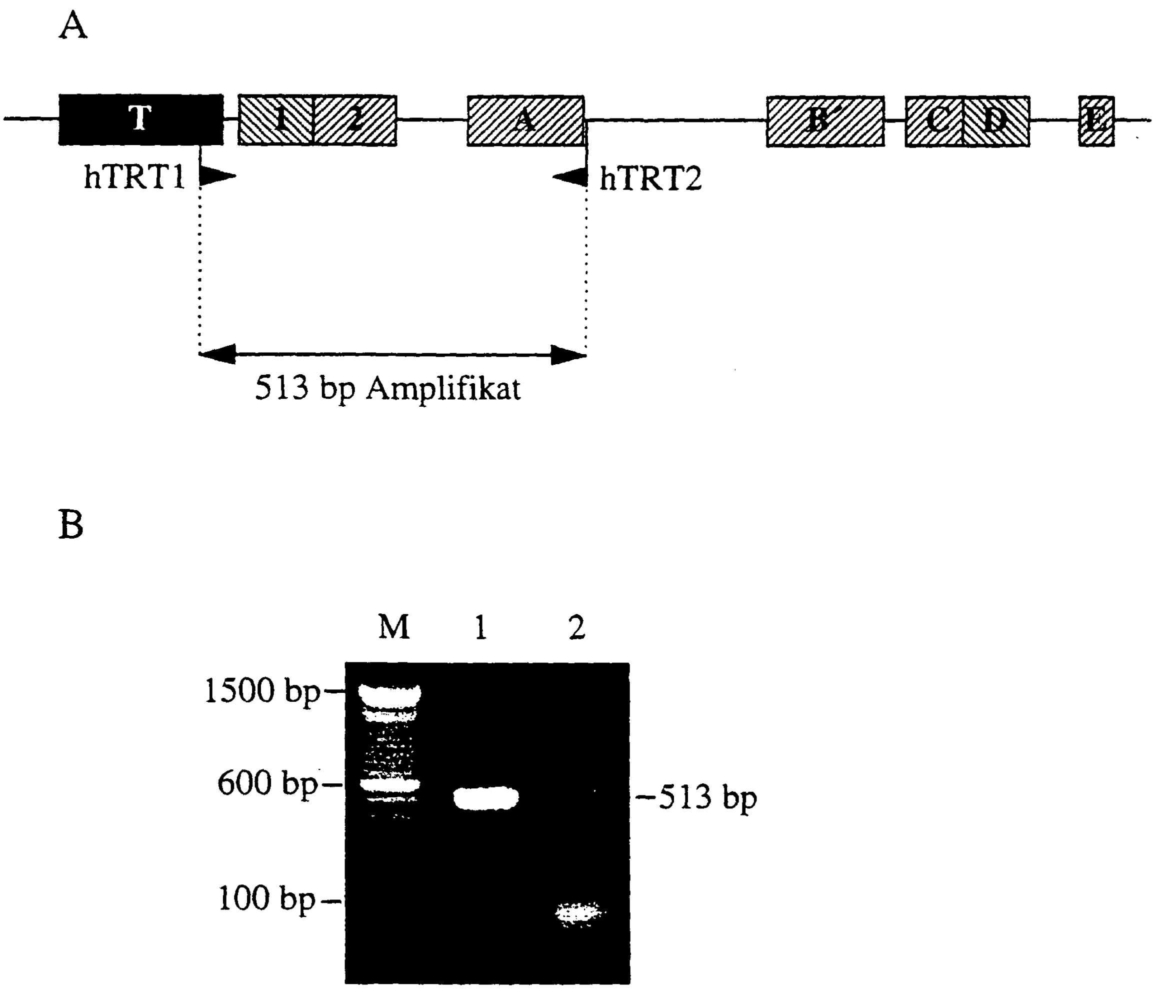
A
T
1
2
A
B'
C
D
E
hTRT1
hTRT2
513 bp Amplifikat
B
M
1
2
1500 bp
600 bp
100 bp
513 bp

**Abb. 2**

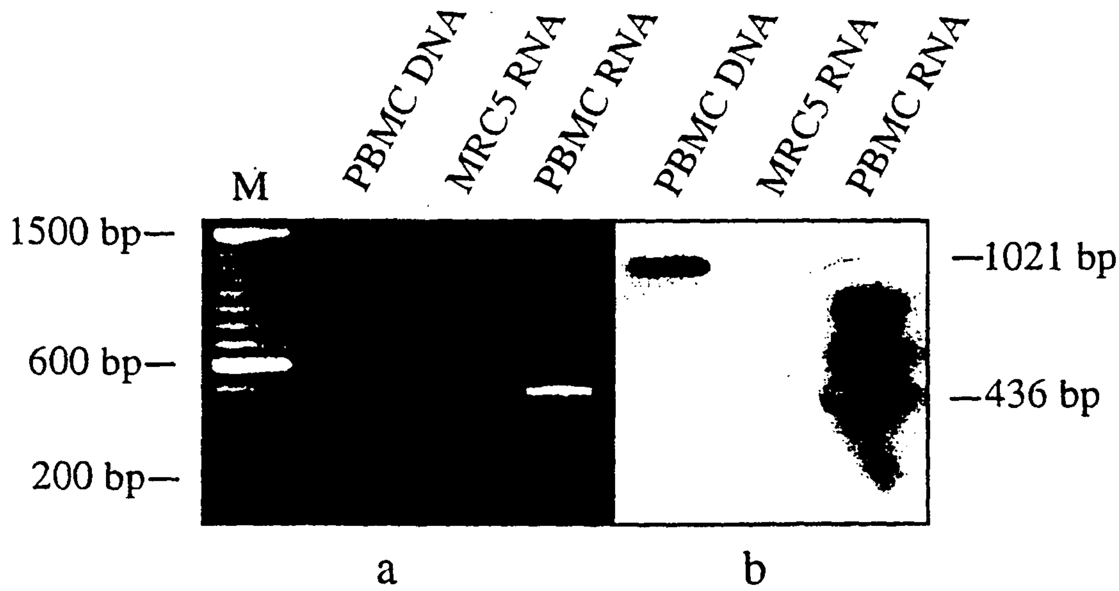
M
PBMC DNA
MRC5 RNA
PBMC RNA
PBMC DNA
MRC5 RNA
PBMC RNA
1500 bp—
600 bp—
200 bp—
—1021 bp
—436 bp
a
b

**Abb. 3**

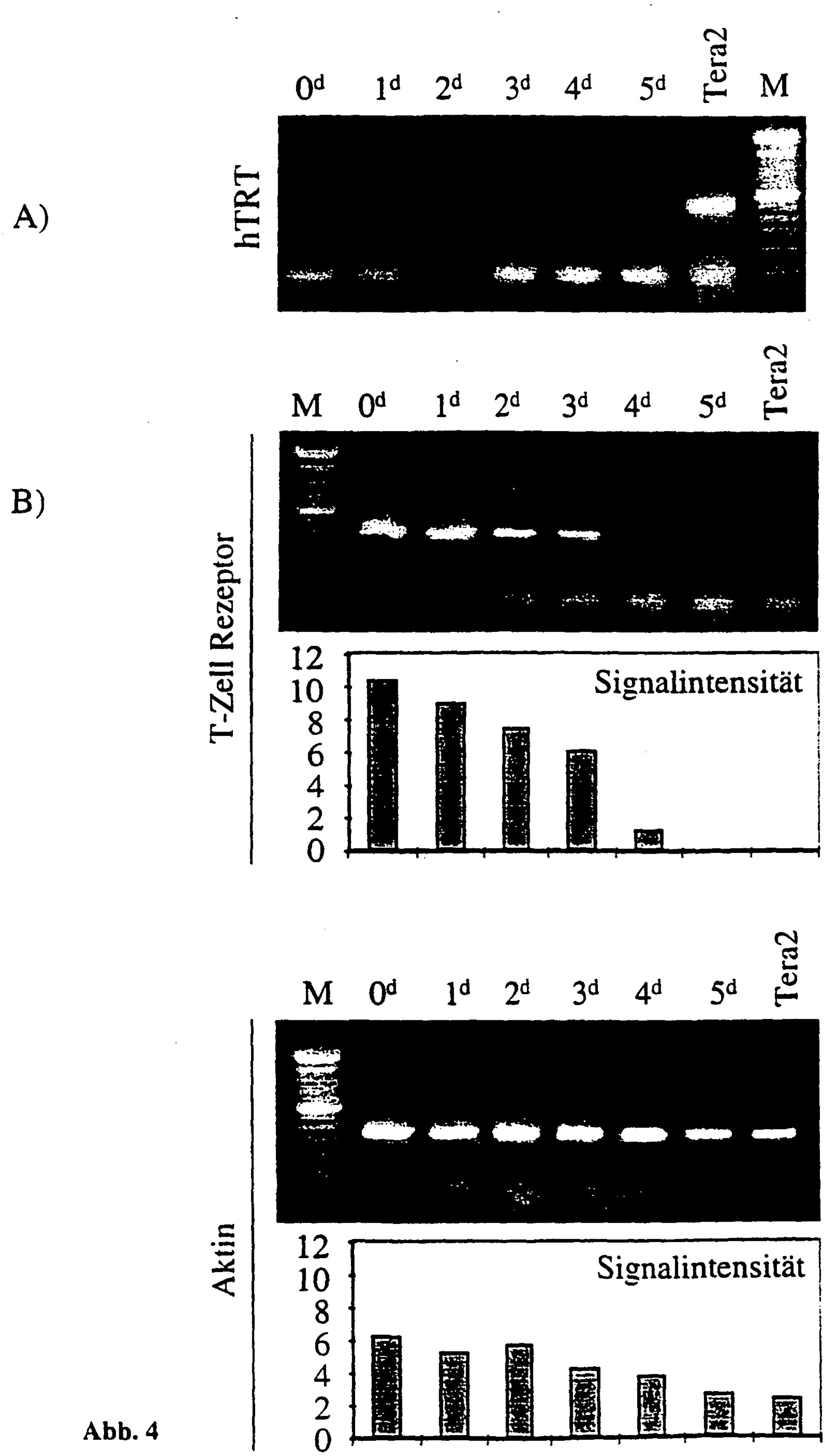
A)
B)
hTRT
0d
1d
2d
3d
4d
5d
Tera2
M
T-Zell Rezeptor
Signalintensität
12
10
8
6
4
2
0
Aktin
Signalintensität

Abb. 4

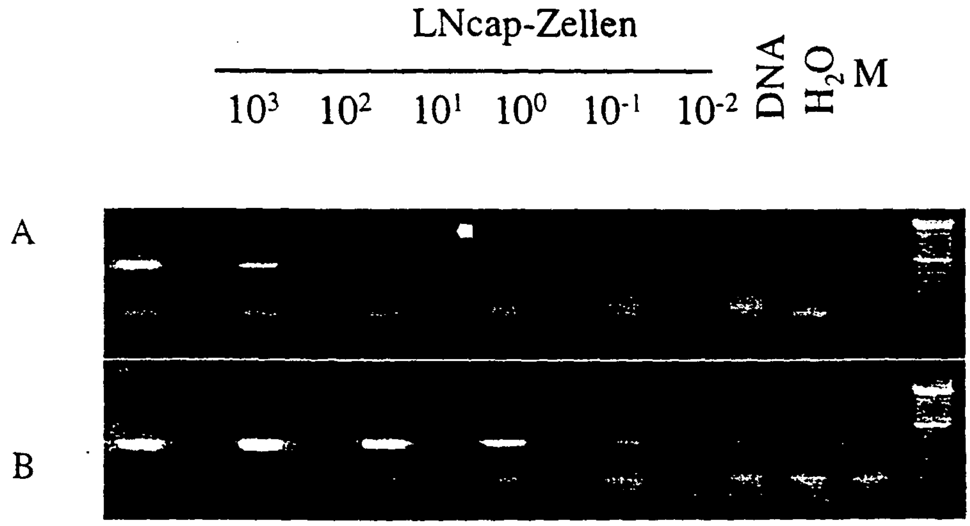


**Abb. 5**

Katalytische Untereinheit der menschlichen Telomerase:

| Name | Sequenz (5`-3) |
|---|---|
| hTRT 1 | CTACCGGAAGAGTGTCTGGAGCAAGTTGCAAAGC |
| hTRT 2 | GGCATACCGACGCACGCAGTACGTGTTCTG |
| hTRT o | CGTTCTGGCTCCCACGACGTAGTC |

T-Zell-Rezeptor:

| Name | Sequenz (5`-3) |
|---|---|
| TCR 1 | GAGGTCGCTGTGTTTGAGCCATCAGAAG |
| TCR 2 | GATCTCATAGAGGATGGTGGCAGACAG |

β-Aktin:

| Name | Sequenz (5`-3) |
|---|---|
| Act 1 | GATGATGATATCGCCGCGCTCGTC |
| Act 2 | CTCAAACATGATCTGGGTCATCTTC |

β-Globin:

| Name | Sequenz (5`-3) |
|---|---|
| Glob 1 | ACCCAGAGGTTCTTTGAGTC |
| Glob 2 | TCTGATAGGCAGCCTGCACT |

**Abb. 6**

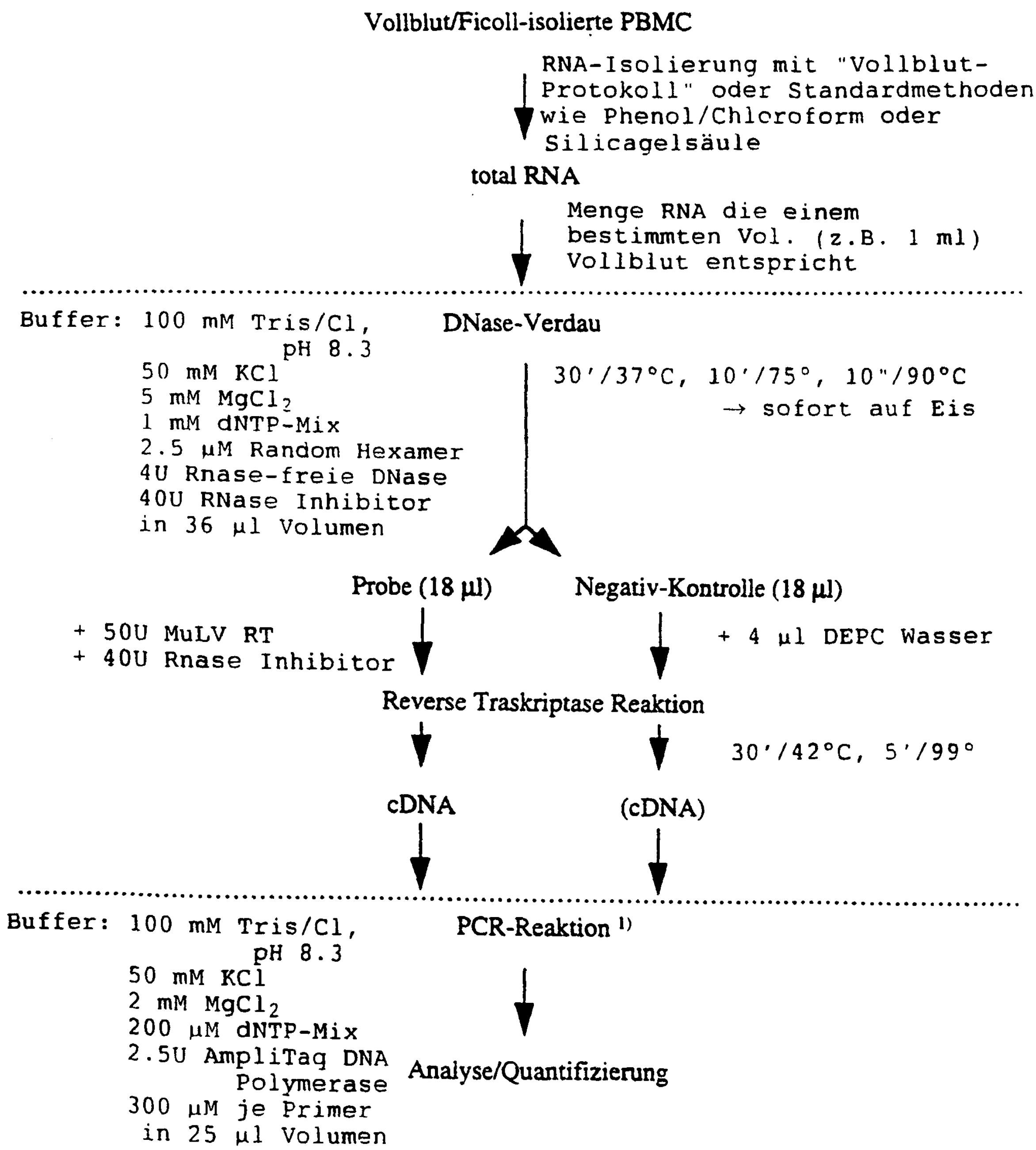


1) PCR-Konditionen:

15"/97°C(15"/97°C,30"/70°C,-0.5°C/cycle,30"/72°C)x10
(15"/94°C,30"/65°C,-0.5°C/cycle,30"/72°C)x20
(15"/94°C,30"/50°C,30"(ext.15"/cycle)/72°C)x10
7'/72°C

**Abb. 7**

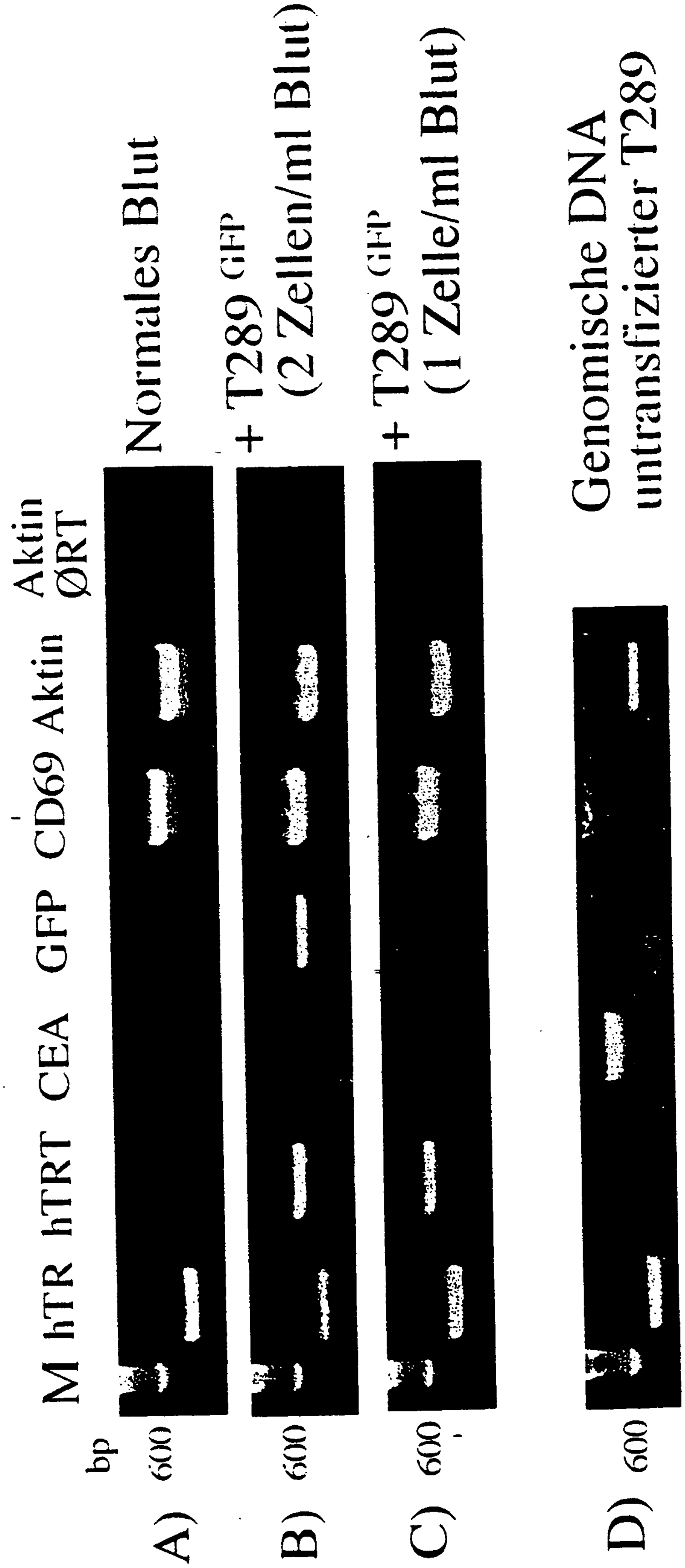
M hTR hTRT CEA GFP CD69 Aktin Aktin ØRT
bp
A) 600
Normales Blut
B) 600
+ T289 GFP (2 Zellen/ml Blut)
C) 600
+ T289 GFP (1 Zelle/ml Blut)
D) 600
Genomische DNA untransfizierter T289

Abb. 8

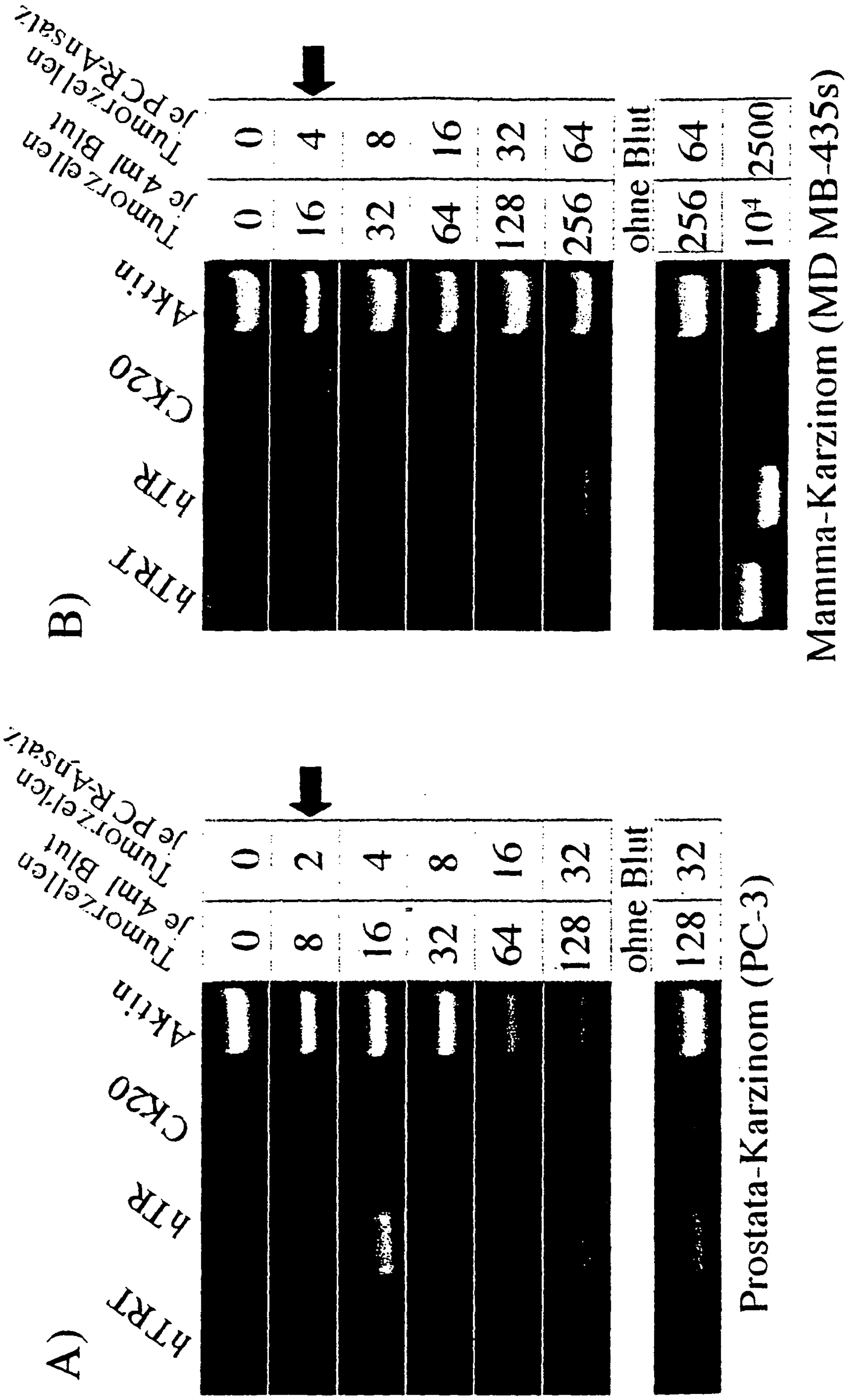
B)
hTRT
hTR
CK20
Aktin
Tumorzellen je 4ml Blut
0
16
32
64
128
256
ohne Blut
256
10^4
Tumorzellen je PCR-Ansatz
0
4
8
16
32
64
64
2500
Mamma-Karzinom (MD MB-435s)
A)
hTRT
hTR
CK20
Aktin
Tumorzellen je 4ml Blut
0
8
16
32
64
128
ohne Blut
128
Tumorzellen je PCR-Ansatz
0
2
4
8
16
32
32
Prostata-Karzinom (PC-3)

Abb. 9

A)

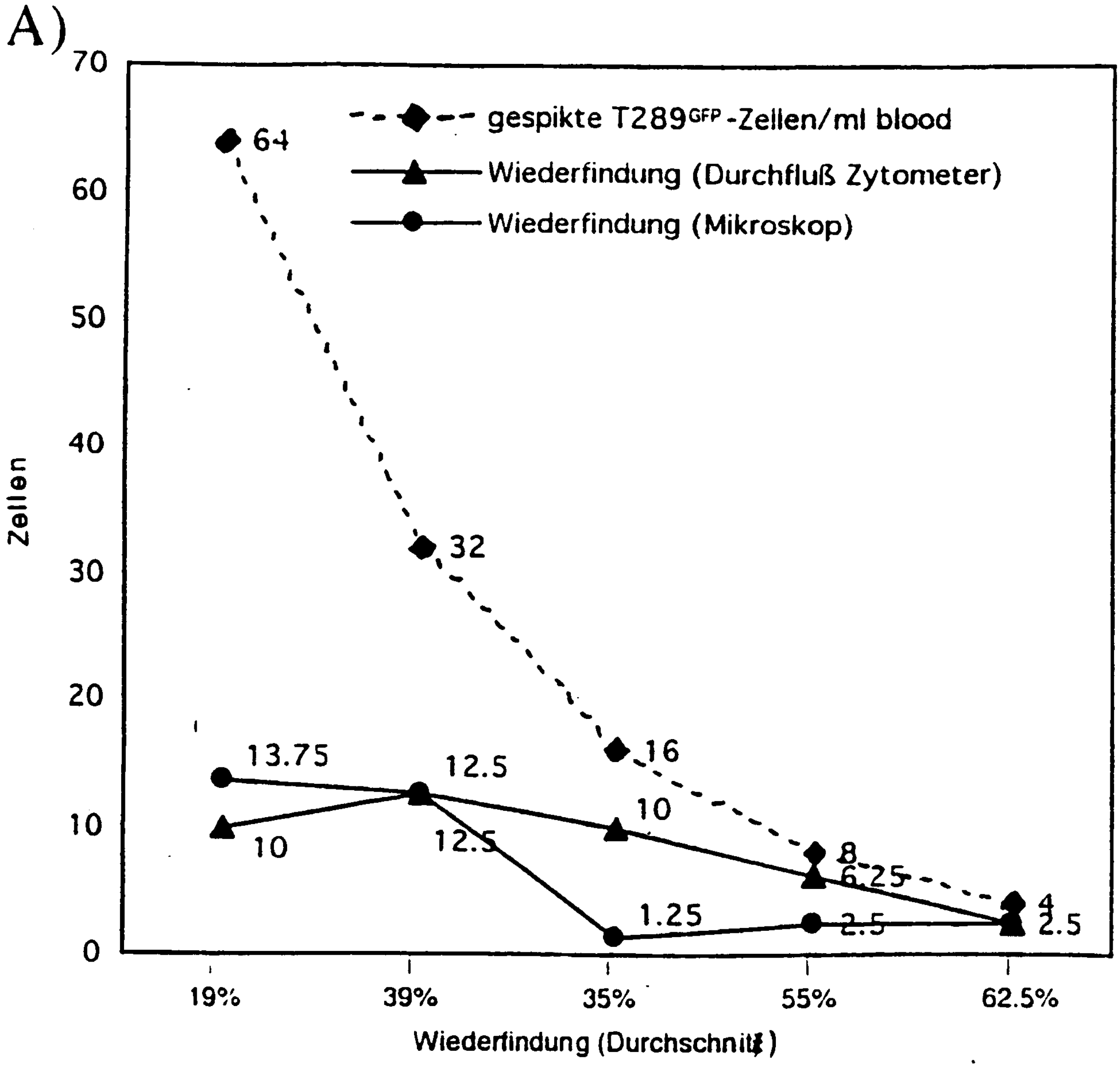

70
60
50
40
30
20
10
0
Zellen
gespikte T289GFP-Zellen/ml blood
Wiederfindung (Durchfluß Zytometer)
Wiederfindung (Mikroskop)
64
32
16
8
4
13.75
12.5
10
12.5
10
1.25
6.25
2.5
2.5
19%
39%
35%
55%
62.5%
Wiederfindung (Durchschnitt)


Abb. 10

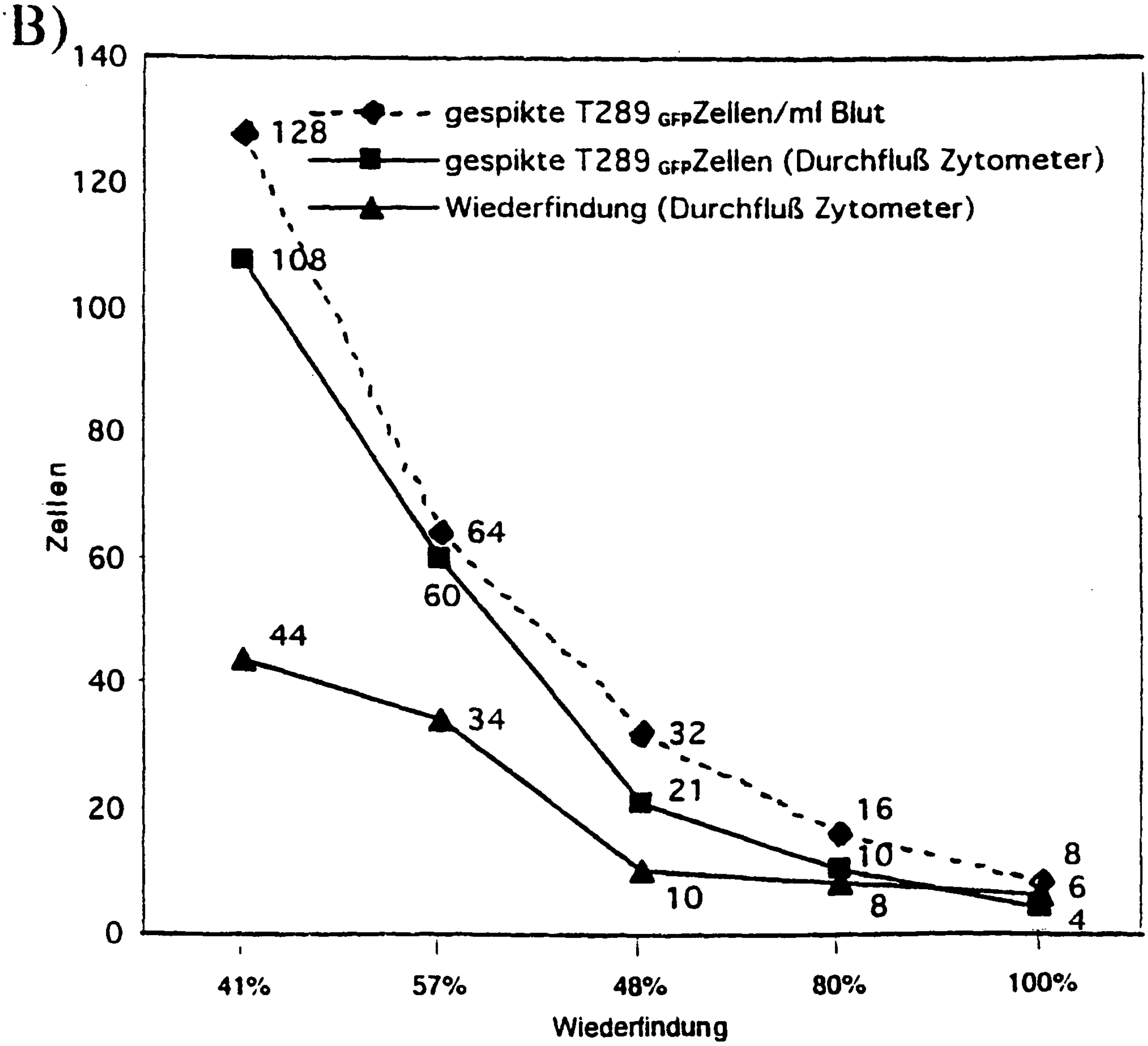
B)
140
120
100
80
60
40
20
0
Zellen
gespikte T289 GFPZellen/ml Blut
gespikte T289 GFPZellen (Durchfluß Zytometer)
Wiederfindung (Durchfluß Zytometer)
128
108
64
60
44
34
32
21
10
16
10
8
8
6
4
41%
57%
48%
80%
100%
Wiederfindung

Abb. 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9802581 A
- WO 9718322 A
- WO 9601835 A
- WO 9007641 A
- US 4683195 A
- US 4683202 A
- US 4965188 A
- US 4889818 A
- GB 2260811 A


### In der Beschreibung aufgeführte Nicht-Patentliteratur


- **Shippen-Lentz et al.** *Science,* 1990, vol. 247, 546
- **Greider et al.** *Nature,* 1989, vol. 337, 331
- **Mehle et al.** *Cancer Res,* 1994, vol. 54, 236
- **Ohyashiki et al.** *Cancer Genet Cytogenet,* 1994, vol. 78, 64
- **Rogalla et al.** *Cancer Genet Cytogenet,* 1994, vol. 77, 19
- **Schwartz et al.** *Cancer,* 1995, vol. 75, 1094
- **Hiyama et al.** *Oncogene,* 1995, vol. 10, 937
- **Shirotani et al.** *Lung Cancer,* 1995, vol. 11, 29
- **Chadeneau et al.** *Cancer Res,* 1995, vol. 55 (2533
- **Counter et al.** *Blood,* 1995, vol. 85, 2315
- **Feng et al.** *Science,* 1995, vol. 269, 1236
- **Nakamura TM ; Morin GB ; Chapman KB ; Weinrich SL ; Andrews WH ; Lingner J ; Harley CB ; Cech TR.** Telomerase catalytic subunit homologs from fission yeast and human. *Science,* 1997, vol. 277, 955-9
- **Meyerson M ; Counter CM ; Eaton EN ; Ellisen LW ; Steiner P ; Caddle SD ; Ziaugra L ; Beijersbergen RL ; Davidoff MJ ; Liu Q.** hEST2, the putative human telomerase catalytic subunit gene, is up-regulated in tumor cells and during immortalization. *Cell,* 1997, vol. 90, 785-95
- **Kim et al.** *Science,* 1994, vol. 266, 2011
- **Chomczynski et al.** *Anal. Biochem.,* 1987, vol. 162, 156
- **Boom et al.** *J. Clin. Microbiol.,* 1990, vol. 29, 495
- **Sambrook et al.** Molecular Cloning: A Laboratory Manual. Cold Spring Harbor Laboratory, 1989
- **Matteucci et al.** *J. Am. Chem. Soc.,* 1981, vol. 103, 3185-3191
- **Guatelli et al.** *Proc. Natl. Acad. Sci. USA,* 1990, vol. 87, 1874-1878
- **Kievits et al.** *J. Virol. Methods,* 1991, vol. 35, 273-286
- **van Gemen et al.** *J. Virol. Methods,* 1994, vol. 49, 157-168
- **van Gemen et al.** *J. Virol. Methods,* 1993, vol. 43, 177-188
- **Higuchi, R.** *Nucleic Acid Res,* 1988, vol. 16, 7351-7367
- **Nakajima-Iiji, S. ; Hamada, H. ; Reddy, P. ; Kakanuga, T.** Molecular structure of the cytoplasmatic ß-actin gene: Interspezies homology of sequences in the introns. *Proc Natl Acad Sci USA,* 1985, vol. 82, 6133-7
- **Toyonaga, B. ; Yoshikai, Y. ; Vadasz, V. ; Chin, B. ; Mak, T.W.** Organization and sequences of the diversity, joining, and constant region of the human T-cell receptor β chain. *Proc Natl Acad Sci USA,* 1985, vol. 82, 8624-8
- **Göttlinger ; Radbruch.** *mta,* 1993, vol. 8 (5), 530-536
- **Kato ; Radbruch.** *Cytometry,* 1993, vol. 14, 384-392
- **J.A. Fleming et al.** *J. clin. Path.,* 1967, vol. 20, 145
- *BCSH Blood Transufsion Task. Transfus. Med.,* 1994, vol. 4, 165-72
- **Koop, S. et al.** *Cancer Res.,* 1995, vol. 55, 2520-2523
- **Chomczynski et al.** *Anal Biochem,* 1987, vol. 162, 156
- **Nakamura et al.** *Science,* 1997, vol. 277, 955-9
- **Altschul, S. F. et al.** *J Mol Biol,* 1990, vol. 215, 403-410
- **K. Yashima et al.** *J. Clin. Pathol.,* 1997, vol. 50, 110-7